(19) **Europäisches Patentamt European Patent Office Office européen des brevets**

(11) **EP 1 261 621 B1**

(12) **EUROPEAN PATENT SPECIFICATION**

(45) Date of publication and mention
of the grant of the patent:
**26.01.2011 Bulletin 2011/04**

(51) Int Cl.:
*G01N 33/53* (2006.01)    *G01N 33/566* (2006.01)
*C07K 7/06* (2006.01)    *C12Q 1/48* (2006.01)

(21) Application number: **00941332.9**

(22) Date of filing: **09.06.2000**

(86) International application number:
**PCT/US2000/016025**

(87) International publication number:
**WO 2000/075167 (14.12.2000 Gazette 2000/50)**

(54) **PHOSPHORYLATION ASSAYS**

TESTVERFAHREN ZUR MESSUNG DER PHOSPHORYLIERUNG

BIOANALYSES REALISEES AU COURS DE LA PHOSPHORYLATION

(84) Designated Contracting States:
**AT BE CH CY DE DK ES FI FR GB GR IE IT LI LU MC NL PT SE**

(30) Priority: **09.06.1999 US 138311 P**
**10.06.1999 US 138438 P**
**08.07.1999 US 349733**
**28.04.2000 US 200594 P**

(43) Date of publication of application:
**04.12.2002 Bulletin 2002/49**

(73) Proprietor: **Molecular Devices, Inc Sunnyvale, CA 94089 (US)**

(72) Inventors:
• **SPORTSMAN, J., Richard**
**Palo Alto, CA 94306 (US)**
• **HOEKSTRA, Merl, F.**
**Cardiff by the Sea, CA 92007 (US)**
• **LEE, Sandra, K.**
**Pacifica, CA 94044 (US)**
• **CAIRNS, Nicholas**
**San Mateo, CA 94402 (US)**
• **KAUVAR, Lawrence, M.**
**San Francisco, CA 94115 (US)**
• **WEI, Huang**
**Santa Clara, CA 95050 (US)**

(74) Representative: **Harrison Goddard Foote**
**Belgrave Hall**
**Belgrave Street**
**Leeds**
**LS2 8DD (GB)**

(56) References cited:
WO-A-00/75332      WO-A1-00/47693
WO-A1-00/47693      WO-A2-00/23785
WO-A2-00/23785      US-A- 5 235 039
US-A- 5 952 236

• JENY STABLES ET AL: "A bioluminescence assay for agonist activity at Potentially Any G-Protein Coupled Receptor" ANALYTICAL BIOCHEMISTRY, vol. 252, 1997, pages 115-126, XP002192656
• JAMESON D M ET AL: "FLUORESCENCE ANISOTROPY APPLIED TO BIOMOLECULAR INTERACTIONS" METHODS IN ENZYMOLOGY, ACADEMIC PRESS INC, SAN DIEGO, CA, US, vol. 246, 1995, pages 283-300, XP002934643 ISSN: 0076-6879
• WAGA I ET AL: "Micro-trap phosphorylation assay of mitogen-activated protein (MAP) kinases to detect their activation by lipopolysaccharides" JOURNAL OF IMMUNOLOGICAL METHODS, ELSEVIER SCIENCE PUBLISHERS B.V.,AMSTERDAM, NL, vol. 190, no. 1, 28 March 1996 (1996-03-28), pages 71-77, XP004020890 ISSN: 0022-1759
• JAMESON ET AL.: 'Fluorescence anisotropy applied to biomolecular interactions' METHODS IN ENZYMOLOGY vol. 246, 1999, pages 283 - 300, XP002934643

**Description**

The following U.S. patent application may assist the reader in understanding the teachings of the present invention: Serial No. 09/160,533, filed September 24, 1998 (Patent No. 6,097,025).

PCT applications which may assist the reader in understanding the teachings of the present invention include:: Serial No. PCT/US99/01656, filed January 25, 1999 (Publication No. WO/1999/037203); Serial No. PCT/US99/03678, filed February 19, 1999 (Publication No. WO/1999/042817); Serial No. PCT/US99/08410, filed April 16, 1999 (Publication No. WO/1999/054711); Serial No. PCT/US99/16057, filed July 15, 1999 (Publication No. WO/2000/004364); Serial No.PCT/US99/16453, filed July 21, 1999 (Publication No. WO/2000/005336); Serial No. PCT/US99/16621, filed July 23, 1999 (Publication No. WO/2000/006991); Serial No. PCT/US99/16286, filed July 26, 1999 (Publication No. WO/ 2000/006989; Serial No. PCT/US99/16287, filed July 26, 1999 (Publication No. WO/2000/006990); and Serial No. PCT/US99/24707, filed October 19, 1999 (Publication No. WO/2000/023785).

Additionally, the following publications may assist the reader in understanding the teachings of the present invention: K.E. van Holde, Physical Biochemistry (2nd ed. 1985); William Bains, Biotechnology from A to Z (1993); Richard P. Haugland, Handbook of Fluorescent Probes and Research Chemicals (6th ed. 1996); Joseph R. Lakowicz, Principles of Fluorescence Spectroscopy (2nd ed. 1999); Bob Sinclair, Everything's Great When It Sits on a Chip: A Bright Future for DNA Arrays, 13 THE SCIENTIST, May 24, 1999, at 18; and Charles R. Cantor and Paul R. Schimmel, Biophysical Chemistry (1980).

## Field of the Invention

The invention relates to phosphorylation. More particularly, the invention relates to luminescence-based assays for detecting phosphorylation and dephosphorylation modifications of proteins, along with the presence, activity, and modulation of enzymes effecting these modifications, including kinases and phosphatases, respectively.

## Background of the Invention

Cellular physiology may be regulated by a variety of mechanisms. In multicellular organisms, these mechanisms may involve cell-signaling pathways in which signal substances are released by one cell to influence the position, nature, and activity of other cells, for example, through intracellular phosphorylation and dephosphorylation reactions.

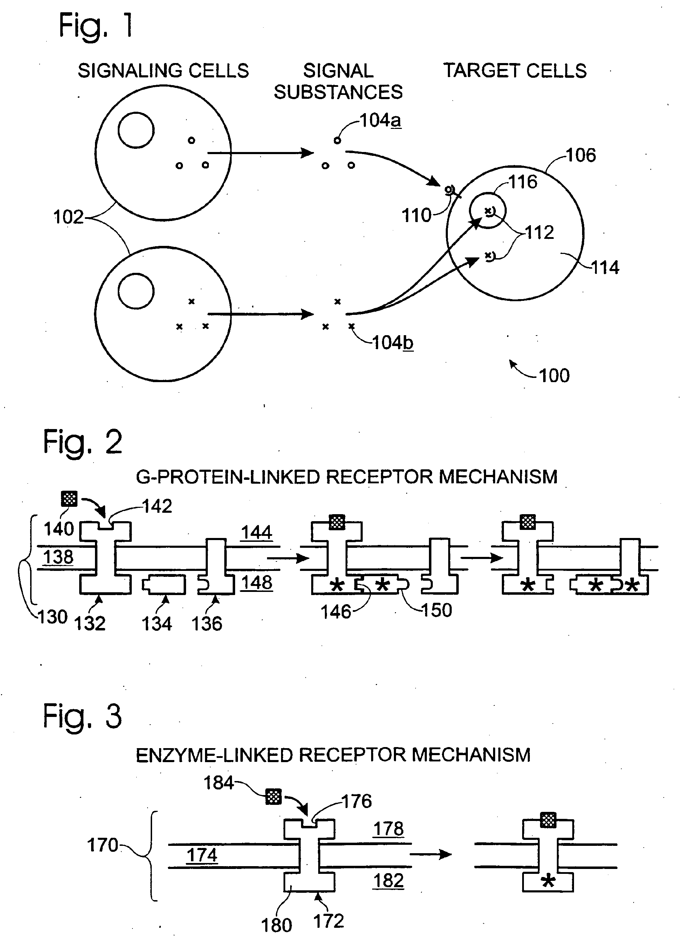

Figure 1 is a schematic view of a representative cell-signaling pathway 100. Here, signaling cells 102 produce signal substances 104a,b that interact with target cells 106 to effect a response in the target cells. These responses may be short term, such as glycogen breakdown or muscle contraction, among others. These responses also may be long term, such as growth, differentiation, reproduction, and/or apoptosis, among others. Generally, these responses are brought about by increasing, decreasing, or maintaining enzyme activity in the target cells.

Signaling cells 102 are cells capable of producing a signal (substance) that can effect a specific response in another (target) cell. The signaling cells may be components of an endocrine, paracrine, or nervous system. The endocrine system is an organism-wide control system that regulates body function using hormones released by endocrine organs into the bloodstream. The endocrine organs include the pituitary gland, thyroid gland, parathyroid glands, adrenal glands, thymus gland, pineal body, pancreas, ovaries, testes, and kidneys. The paracrine system is a local control system that regulates nearby cells using local mediators released into the extracellular medium. The nervous system is a specialized control system that regulates specific cells using electrical impulses and neurotransmitters.

Signal substances 104a,b are substances through which a signaling cell may communicate with target cells, evoking a specific response. Signal substances may act as hormones, local mediators, and/or neurotransmitters, among others. Signal substances may take the form of proteins, small peptides, amino acids, nucleotides, steroids (e.g., cortisol, steroid sex hormones, vitamin D), retinoids, fatty acid derivatives, and dissolved gases (e.g., nitric oxide (NO) and carbon monoxide (CO)), among others.

Target cells 106 are cells capable of responding to a specific signal substance produced by a signaling cell. The ability to respond may depend on the cell and on the signal substance. For example, the signal substance thyroxine from the thyroid gland may evoke a response in nearly all cells, whereas the signal substance progesterone from the ovary may evoke a response only in specific cells in the lining of the uterus. The target response may include kinase activity, GTP binding, and/or cyclic nucleotide production.

The ability of a cell to respond to a given signal substance generally is determined by whether the cell includes a receptor for the signal substance. Here, a receptor is any molecule or supramolecular assembly capable of specifically binding a signal substance and initiating a response in a target cell. Representative receptors include cell-surface receptors 110 located on the surface of the target cell and intracellular receptors 112 located within the cytosol 114 or nucleus 116 of the target cell.

The nature of the response initiated by binding of a signal substance is determined by the intracellular machinery to which the receptor is operatively coupled. For example, binding of the neurotransmitter acetylcholine to identical receptors in heart muscle cells and secretory cells causes muscle relaxation in the heart muscle cells and secretion in the secretory cells, due to differences in the associated intracellular machinery.

The remainder of this section examines (1) the receptor mechanisms that cells use to bind signal substances and to communicate this binding to the cell interior, (2) the intracellular pathways that cells use for regulation, and (3) the effects of errors in cell-signaling pathways.

## 1. Receptor Mechanisms

Target cells generally have receptors capable of specifically binding specific signal substances, including cell-surface receptors and/or intracellular receptors, as described above. Cell-surface receptors are more common and include (A) G-protein-linked receptors, (B) enzyme-linked receptors, and (C) ion-channel-linked receptors. These receptors typically bind large and/or watersoluble signal substances, such as many peptide hormones. Intracellular receptors are less common and include (A) guanylyl cyclase and (B) ligand-activated gene regulatory proteins. These receptors typically bind small and/or water-insoluble signal substances, such as steroid hormones, thyroid hormones, retinoids, vitamin D, and NO.

**[0014]** Figure 2 is a schematic view of a representative G-protein-linked cell-surface receptor mechanism 130 that includes a receptor protein 132, a G-protein 134, and a target protein 136. These proteins may be positioned on or within the plasma membrane 138 of a target cell. In use, a specific signal substance 140 binds to a signal-substance binding site 142 on the extracellular side 144 of the receptor protein and thereby creates, exposes, or otherwise activates (*) a G-protein binding site 146 on the intracellular side 148 of the receptor protein. The G-protein then binds to the G-protein binding site on the receptor protein and thereby creates, exposes, or otherwise activates (*) a target-protein binding site 150 on the G-protein. The G-protein then dissociates from the receptor protein, binds (via the target-protein binding site) to the target protein, and activates (*) the target protein. Activation and deactivation of the G-protein may involve binding of a guanosine triphosphate (GTP) molecule and dephosphorylation of the GTP molecule, respectively. The receptor protein may belong to a large superfamily of homologous, seven-pass transmembrane proteins. These seven-pass proteins consist of a single polypeptide chain that crosses the membrane seven times, with an extracellular signal-substance binding portion and an intracellular catalytic portion. The G-protein may be trimeric, consisting of three polypeptide chains--$\alpha$, $\beta$, and $\gamma$-that associate and dissociate during signaling. The target protein may consist of an enzyme or ion channel, among others. In particular, the target protein may be an enzyme that modulates the presence or activity of second messengers within the cell. These second messengers (also known as intracellular messengers or intracellular mediators) may bind allosterically to specific cellular proteins to alter their conformation and hence their activity. These second messengers include adenosine 3',5'-cyclic monophosphate (cAMP) and calcium ($Ca^{2+}$).

In the cAMP pathway, the target protein may be adenylyl cyclase (also known as adenylate cyclase), and the G-protein may be a stimulatory G-protein ($G_s$) that activates the adenylyl cyclase to make cAMP, or an inhibitory G protein ($G_i$) that inhibits the adenylyl cyclase to prevent it from making CAMP. The cAMP produced by the adenylyl cyclase activates cAMP-dependent protein kinase (A-kinase), which is a serine/threonine kinase that in turn activates or inhibits other enzymes to effect a physiological response. For example, in connection with glycogen metabolism, A-kinase may inhibit glycogen synthase to shut down glycogen synthesis, and simultaneously activate phosphorylase kinase that in turn activates glycogen phosphorylase to break down glycogen. A variety of signal substances use cAMP as a second messenger, including calcitonin, chorionic gonadotropin, corticotropin, epinephrine, follicle-stimulating hormone, glucagon, luteinizing hormone, lipotropin, melanocyte-stimulating hormone, norepinephrine, parathyroid hormone (PTH), thyroid-stimulating hormone, and vasopressin. The level of cAMP is reduced by phosphodiesterases, and the activity of kinases is reversed by phosphatases, as described below.

In the $Ca^{2+}$ pathway, the target protein may be a phospholipase with specificity for a phosphoinositide (i.e., inositol phospholipid), and the G-protein may be $G_q$, which activates the phospholipase to cleave the phosphoinositide to produce an intermediate that releases $Ca^{2+}$ from the endoplasmic reticulum. For example, the phospholipase phosphoinositide-specific phospholipase C (phospholipase C-$\beta$) cleaves the phosphoinositide phosphatidylinositol 4,5-bisphosphate ($PIP_2$) to produce the second messengers inositol triphosphate ($IP_3$) and diacylglycerol. The inositol triphosphate is water soluble and diffuses to the endoplasmic reticulum (ER), where it releases $Ca^{2+}$ from the ER by binding to $IP_3$-gated $Ca^{2+}$-release channels in the ER membrane. The diacylglycerol is membrane bound and may be cleaved to form the second messenger arachidonic acid or may activate the $Ca^{2+}$-dependent serine/threonine kinase protein kinase C that in turn activates or inhibits other enzymes to effect a response. A variety of signal substances use $Ca^{2+}$ as a second messenger, including acetylcholine, antigen, thrombin, and vasopressin.

Figure 3 is a schematic view of a representative enzyme-linked cell-surface receptor mechanism 170 that includes a receptor protein 172 positioned across the plasma membrane 174 of a target cell. The receptor protein includes a signal-substance binding site 176 on the extracellular side 178 of the membrane and a catalytic portion 180 on the intracellular side 182 of the membrane. (In some cases, the catalytic portion of the receptor may be replaced or augmented by a separate enzyme directly associated with the receptor protein.) In use, a specific signal substance 184 binds to the signal-substance binding site, initiating a series of events (such as dimerization and concomitant autophosphorylation of the receptor proteins) that activates (*) the catalytic portion of the receptor. The receptor protein may belong to one of at least five classes of single-pass transmembrane proteins: (A) receptor guanylyl cyclases, which catalyze the production of guanosine 3',5'-cyclic monophosphate (cGMP) in the cytosol; (B) receptor tyrosine kinases, which phosphorylate specific tyrosine residues on some intracellular proteins, (C) tyrosine-kinase-associated receptors, which associate with proteins that phosphorylate specific tyrosine residues on some intracellular proteins; (D) receptor tyrosine phosphatases, which dephosphorylate specific tyrosine residues on some intracellular proteins, and (E) receptor serine/threonine kinases, which phosphorylate specific serine or threonine residues on some intracellular proteins. Some of these receptors are described below in more detail.

The signal substance also may bind to intracellular receptors, such as guanylyl cyclase. This enzyme produces cGMP from GTP; which then acts as a second messenger much like cAMP. As described above, cGMP also may be produced by enzyme-linked cell-surface receptors. cGMP is present in most tissues at levels 1/10 to 1/100 those of cAMP. A variety of compounds increase cGMP levels in cells, including (1) the hormones acetylcholine, insulin, and oxytocin, (2) the guanylate cyclase stimulators (and vasodilators) nitroprusside, nitroglycerin, sodium nitrate, and nitric oxide, (3) chemicals such as serotonin and histamine, and (4) peptides such as atrial natriuretic peptide (ANP) that relax smooth muscle.

## 2. Intracellular Signaling Pathways

Target cells may have intracellular signaling pathways capable of specifically binding signal substances, including cell-surface receptors and intracellular receptors, as described above. These pathways may include (1) a phosphorylation pathway involving ATP/ADP, and (2) a GTP-binding pathway involving GTP/GDP.

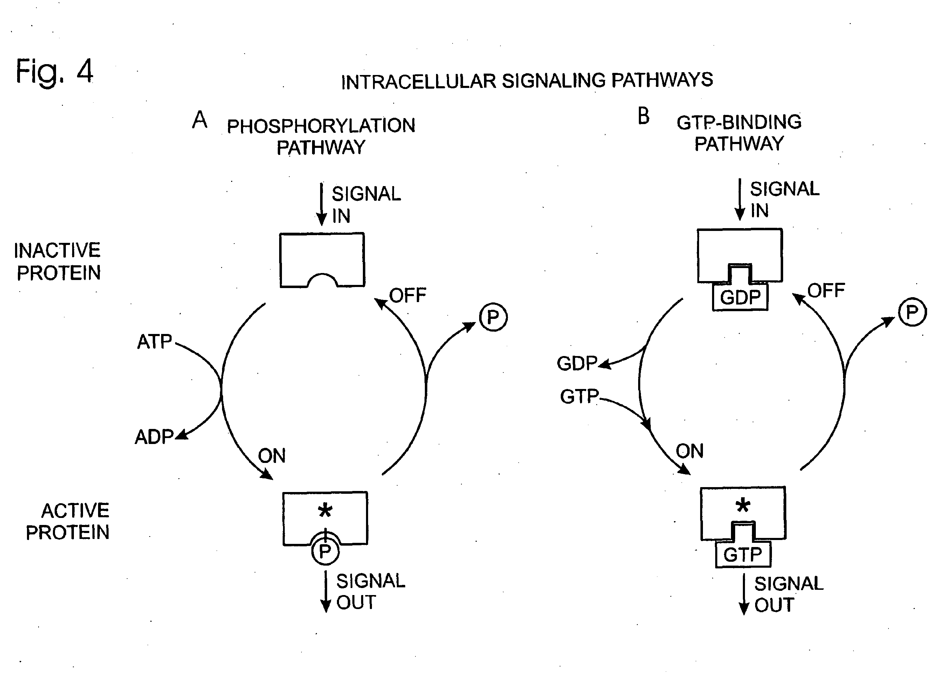

Figure 4A is a schematic view of a representative phosphorylation pathway. Phosphorylation is the predominant mechanism used to regulate protein activity in eucaryotic cells. In phosphorylation, a phosphate group (P) is reversibly attached to the side chain of an amino acid in a protein. The attached phosphate group may cause structural changes in the protein, for example, due to electrostatic interactions between the negative charges on the phosphate group and positive charges on the side chains of nearby amino acids. These structural changes may affect the activity of the phosphorylated protein, enhancing or inhibiting its function.

Specialized enzymes control phosphorylation in cells. In particular, protein kinase enzymes transfer phosphate groups to proteins, and protein phosphatase enzymes remove phosphate groups from proteins. Protein kinases and protein phosphatases are found in great variety in eucaryotic cells: a single cell may contain more than 100 different kinases, and one percent of genes may code for kinases.

There are two major categories of kinases: (1) serine/threonine (S/T) kinases, and (2) tyrosine kinases. The S/T kinases function by selectively phosphorylating serine and threonine side chains on substrate proteins or peptides. These kinases include cyclic AMP-dependent kinase (A-kinase), cyclic GMP-dependent kinase (G-kinase), protein kinase C (C-kinase), $Ca^{2+}$-calmodulin-dependent kinase (CaM-kinase), phosphorylase kinase, MAP kinase, and TGF-β receptor, among others. The S/T kinases are predominantly cytosolic. The tyrosine kinases function by selectively phosphorylating tyrosine side chains on substrate proteins or peptides. These kinases include the receptor kinases for epidermal growth factor (EGF), platelet-derived growth factor (PDGF), fibroblast growth factors (FGFs), hepatocyte growth factor (HGF), insulin, insulinlike growth factor-1 (IGF-1), nerve growth factor (NGF), vascular endothelial growth factor (VEGF), and macrophage colony stimulating factor (M-CSF). These kinases also include the nonreceptor kinases associated with the tyrosine-kinase-associated receptors, such as the Src family (Src, Yes, Fgr, Fyn, Lck, Lyn, Hck, and Blk) and Janus family (JAK1, JAK2, and Tyk2) kinases. The tyrosine kinases are predominantly membrane bound. A few kinases function by selectively phosphorylating threonine and tyrosine side chains on substrate proteins or peptides. These kinases include the mitogen-activated protein (MAP) kinase-kinase.

Figure 4B is a schematic of a representative GTP-binding pathway. The GTP-binding pathway generally resembles the phosphorylation pathway in that each pathway involves transfer of a phosphate group to a protein. However, in the GTP-binding pathway, the protein gains a phosphate group by exchanging a bound GDP for a bound GTP, whereas in the phosphorylation pathway, the protein gains a phosphate group by covalent addition of the phosphate group to a serine, threonine, or tyrosine by a kinase enzyme. The binding of a GTP to a GTP-binding protein may cause structural changes in the protein that in turn affect the activity of the protein. Examples of GTP-binding proteins include the trimeric G-proteins described above and the Ras superfamily of monomeric GTPases. The Ras proteins are activated by release of bound GDP and binding of GTP stimulated by guanine-nucleotide releasing proteins (GNRPs). The Ras proteins are inactivated by hydrolysis of the bound GTP by GTPase-activating proteins (GAPs).

A physiological response may require stimulation by only a single type of signal substance, or may require stimulation by two or more types of signal substances. The latter mechanism permits finer tuning of the physiological response through signal integration. For example, a protein may be activated only by phosphorylation by two different kinases, themselves activated by binding of two different signal substances to two different receptors. Alternatively, a protein may be activated only by concurrent phosphorylation and GTP binding, or by binding of two subunits whose binding is contingent on phosphorylation by separately activated kinases.

## 3. Effects of Errors

Errors in the signal transduction and regulation pathways described above can cause cancer and other diseases. Indeed, a primary cause of cancer is a mutation that makes a stimulatory gene product hyperactive, converting a proto-oncogene into an oncogene. The primary classes of known proto-oncogenes include the following cell-signaling proteins: (1) growth-factor receptors acting via tyrosine kinases, (2) GTP binding proteins, (3) membrane/cytoskeleton-associated tyrosine kinases, (4) cytoplasmic tyrosine kinases, (5) steroid-type growth-factor receptors, and (6) S/T kinases. Consequently, cell-signaling proteins have become important subjects of research and drug development.

Assays that determine the presence and activity of cell-signaling components are important tools for high-throughput screening laboratories. Unfortunately, current assays have a number of shortcomings. For example, the presence and activity of kinases can be determined using assays capable of detecting phosphorylated amino acids. In a standard kinase assay, radioactive ATP and an appropriate protein substrate are added to a sample. If the sample includes kinases, radioactive phosphate groups will be transferred from the radioactive ATP to the protein substrate. The protein substrate and radioactive ATP can be separated, and the presence and activity of kinases determined by assaying the amount of radioactive protein substrate. Unfortunately, this assay involves radioactivity, presenting a short-term safety hazard for the assay operator and a long-term storage and disposal problem. Moreover, the assay is heterogeneous, requiring separation of components for analysis. Significantly, assays for other cell-signaling components may have similar shortcomings, as well as slow time courses and unstable endpoints that require precise timing of assay readouts. Thus, there is a need for improved assays for detecting the presence and activity of cell-signaling components.

## Summary of the Invention

The invention provides luminescence-based assays for detecting phosphorylation and dephosphorylation modifications of proteins, along with the presence, activity, and modulation of enzymes effecting these modifications, such as kinases and phosphatases, respectively.

## Brief Description of the Drawings

Figure 1 is a schematic view of a cell-signaling pathway.
Figure 2 is a schematic view of a G-protein-linked cell-surface receptor mechanism that includes a receptor protein, a G-protein, and a target protein, all associated with the plasma membrane of a target cell.
Figure 3 is a schematic view of an enzyme-linked cell-surface receptor mechanism that includes a receptor protein positioned across the plasma membrane of a target cell.
Figure 4 is a schematic view of two common intracellular signaling pathways: (A) a phosphorylation pathway involving ATP/ADP, and (B) a GTP-binding pathway involving GTP/GDP.
Figure 5 is a schematic view of several assays provided by the invention.
Figure 6 is a diagrammatic representation of one of the assays of Figure 5.
Figure 7 shows the dependence of luminescence polarization on the concentration of antibody available to react with a labeled tracer peptide.
Figure 8 shows the results of an assay in which unlabeled "modified substrate" is able to displace luminescently labeled tracer in binding to antibodies that specifically bind both, in a concentration-dependent manner.
Figure 9 shows a dose-dependent competition assay with labeled tracer, in analogy with Figure 8.
Figure 10 shows a competition assay with a modified substrate generated by the action of the insulin receptor cytoplasmic kinase domain (IR-CKD), plotted as a function of $\mu$L, catalysis mixture [CKD+buffer+ATP+substrate peptide] added to constant amount of antibody and tracer.
Figure 11 shows the use of the assay of Figure 10 to assess kinase activity of IR-CKD, plotted as a function of time for a constant amount of catalysis mixture.
Figure 12 shows the use of the assay of Figure 10 to assess inhibitor activity at a fixed time.
Figure 13 shows the use of the assay of Figure 10 to assess agonist activity with respect to IR-CKD at a fixed time.
Figure 14 shows the use of the invention assay for a CKD different than the CKD of the insulin receptor.
Figure 15 is an HPLC chromatographic trace of fragments of IR-CKD.
Figure 16 shows the use of the competition assay of the invention to map the location of phosphorylated tyrosine in the separated peptides.

Figure 17 is a schematic view of a tyrosine kinase assay.

Figure 18 is a graph showing competition curves for a tyrosine kinase assay in which a mixture of tracer and antibody is added to a simulated enzyme product (o-phospho-L-tyrosine).

Figure 19 is a graph showing competition curves for a tyrosine kinase assay in which a mixture of EDTA, tracer, and antibody is added to the simulated enzyme product of Figure 18.

Figure 20 is a graph of polarization versus phosphotyrosine competitor concentration in 96-well and 1536-well microplates, showing how luminescence polarization may be used in a homogeneous immunoassay format to measure inhibition of enzyme activity.

Figure 21 is a graph of polarization versus phosphotyrosine competitor for the assay of Figure 19, determined for sample volumes of 40, 60, 80, and 100 $\mu$L concentration in 384-well microplates.

Figure 22 shows the effects of incubating 10 nM TK-1 tracer with different concentrations of MM-GA

Figure 23 shows a dose-response curve for ATP, with 10 nM TK-1 tracer and 1.6 nM (estimated) MM-Ga.

Figure 24 shows a dose-response curve for TK-1 calibrator, with 10 nM TK-1 tracer and 1.6 nM (estimated) MM-Ga.

Figure 25 shows binding of a fluorescein-labeled mono-phosphoserine peptide STK-1 tracer to various concentrations of MM-Ga.

Figure 26 shows the effects of HEPES-based buffers on the MM-Ga/phosphopeptide interaction.

Figure 27 shows an endpoint assay of PKA activity with MM-Ga.

Figure 28 shows a time-course assay of PKA activity with MM-Ga performed under the reaction conditions of Figure 27.

Figure 29 shows a time-course assay of PKA activity with a $Ga^{3+}$-coated plate.

Figure 30 shows polarization calibration curves for TK-1 with BODIPY-TMR tracer, for (A) 500 msec and (B) 200 msec integration times.

## Detailed Description

The invention provides luminescence-based assays for detecting phosphorylation and dephosphorylation modifications of proteins, along with the presence, activity, and modulation of enzymes effecting these modifications, including kinases and phosphatases, respectively. These assays may include luminescence polarization and luminescence energy transfer assays, among others. These assays also may include peptide substrates and binding partners specific to phosphorylated or unphosphorylated versions of the substrates, or phosphorylated substrates in general.

Luminescence assays have been described. In a typical format, the ability of an analyte to compete for a specific binding partner with a luminescently labeled form of an analyte or analog is measured in a heterogeneous assay by determining, for example, the level of the luminescent competitor in a solution or attached to a solid support after the competitor and the analyte are contacted with a member of a specific binding pair to which both bind, which member is coupled to the solid support. The amount of luminescent label bound to the solid support is inversely proportional to the concentration of the analyte, while the amount of luminescent label in the solution is directly proportional thereto.

Such an assay is possible in homogeneous form if the luminescence polarization or other measurable property of the luminescent label changes when it is bound to the opposing member of its specific binding pair. If the level of polarization, for example, is enhanced when the luminescent competitor is bound, the degree of polarization will be inversely correlated with the concentration of the tested-for analyte. See, for example, Jameson, D.M., et al., "Fluorescence Anisotropy Applied to Biomolecular Interactions," in Methods in Enzymology (1995) 246:283-300.

Luminescence assays also have been used to detect proteolytic activity where proteolysis liberates a luminescently labeled material. Separation of the liberated luminescent tracer thus measures the proteolytic activity of an enzyme. See U.S. Patent No. 5,235,039.

Recently, an abstract by Seethala, K. published in advance of the meeting of the Society for Bimolecular Screening, to be held September 22-25, 1997, reports the development of a "fluorescence polarization-competition immunoassay ... in which the tyrosine kinase phosphorylated peptide/protein competes with luminescent phosphopeptide used as a tracer in a pY antibody immunocomplex." Here, pY represents phosphoryl tyrosine.

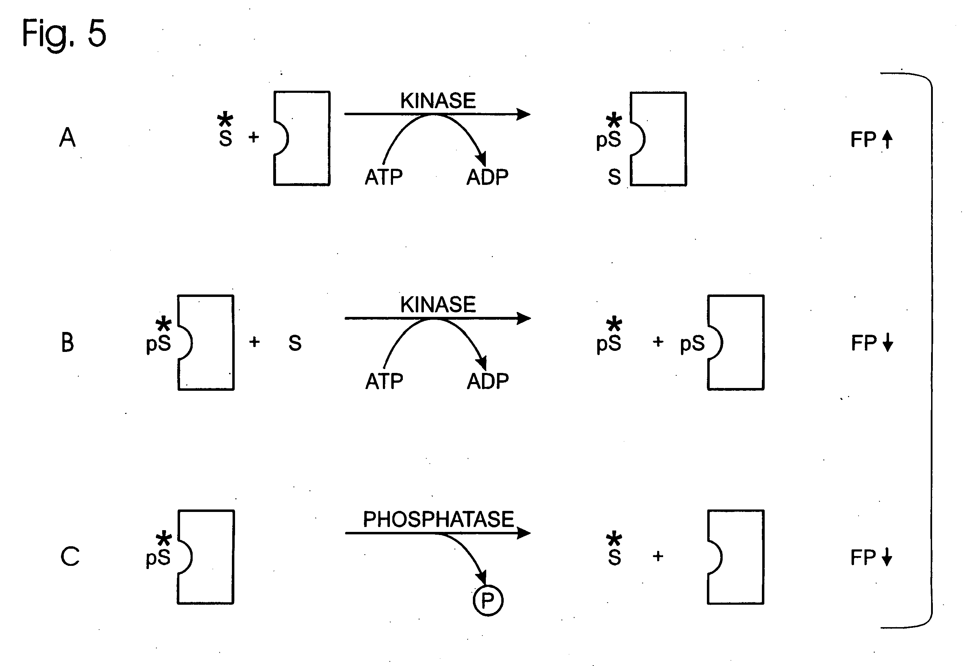

The invention provides assays generally applicable to determining the presence, activity, and/or modulation of proteins effective in making posttranslational modifications of synthesized protein, especially phosphorylation and dephosphorylation modifications at tyrosine, threonine, or serine residues. Figure 5 shows a few of these assays, in particular, luminescence-polarization-based (FP) assays. Here, S is a substrate containing an amino acid such as tyrosine, threonine, or serine capable of being phosphorylated, pS is a phosphorylated version of the substrate, S* and pS* are luminescently labeled versions of S and pS, respectively, and the substantially rectangular box is a binding partner capable of recognizing pS and pS*, but not S or S*. Panel A shows a direct kinase assay, in which S* is phosphorylated by a kinase to yield pS*, which binds to the binding partner, leading to an increase in polarization. Panel B shows an (indirect) competition kinase assay, in which S (typically present in excess of pS*) is phosphorylated by a kinase to yield pS, which competes off pS* for binding to the binding partner, leading to a decrease in polarization. Panel C shows a direct phosphatase assay, in which pS* is dephosphorylated by a phosphatase to yield S*, which does not bind to the binding partner, leading to a decrease in polarization. Analogous assays can be constructed using a binding partner that recognizes S and S*, but not pS or pS*, namely, a direct assay for kinase activity and direct and (indirect) competition assays for phosphatase activity.

The invention also provides assays that can be performed in a homogeneous environment. The assays may involve competition of a luminescent tracer for an opposing member of a specific binding pair, such as an antibody. In these assays, the tracer competes with a modified form of a substrate where the modification may include any posttranslational modification of a protein. The concentration of the modified substrate may be used as an index to the activity of the enzyme catalyzing the modification, or as an index to an agonist with regard to that activity or an inhibitor with regard to that activity, or it may be used to map sites of modification following fragmentation of the protein by physical or enzymatic means. If the enzyme is associated with a receptor, the assay effectively measures the ability of a test compound to act as an agonist or antagonist of the receptor, modulating activity of the receptor. Thus, the assays of the invention provide a broad spectrum of assessment to evaluate metabolism, protein synthesis, and catalytic activities.

Thus the invention relates to a method to determine the concentration of a modified substrate which method comprises contacting a sample containing the modified substrate with a luminescent tracer, wherein the luminescent tracer and the modified substrate compete for binding to a substance, followed by measuring the luminescence polarization in the sample. When the tracer is bound to the substance, its luminescence polarization is changed from the value associated with the unbound form of the luminescent tracer.

In one aspect, the invention provides a method of detecting addition or removal of a phosphate group to or from a substrate comprising: contacting a luminescent peptide with a binding partner that binds specifically to a phosphorylated peptide substantially without regard to the particular amino acid sequence of the peptide, wherein the peptide is a substrate for an enzyme that catalyzes addition or cleavage of a phosphate group to or from a protein, and measuring luminescence polarization from the luminescent peptide, wherein the amount of measured luminescence polarization can be related to the extent of binding between the luminescent peptide and the binding partner.

In another aspect, the invention provides a method of detecting addition or removal of a phosphate group to or from a substrate comprising: contacting a luminescent peptide with a binding partner that binds specifically to a phosphorylated peptide substantially without regard to the particular amino acid sequence of the peptide, wherein the peptide is a substrate for an enzyme that catalyzes addition or cleavage of a phosphate group to or from a protein, and measuring luminescence resonance energy transfer (FRET), and relating FRET to the extent of binding between the luminescent peptide and the binding partner.

In another aspect, the invention provides a method to assess the ability of a candidate compound to enhance or inhibit phosphorylation in a substrate protein which method comprises: performing the method of any preceding claim in the presence and absence of said candidate compound, and determining the degree of polarization or, as the case may be, the luminescence resonance energy transfer (FRET) in the presence as compared to the absence of said candidate compound, whereby an increase in the degree of measured polarization or, as the case may be, the luminescence resonance energy transfer (FRET) in the presence as opposed to the absence of said candidate compound identifies said compound as an inhibitor of said phosphorylation, and whereby a decrease in the degree of polarization or, as the case may be, the luminescence resonance energy transfer (FRET) in the presence as compared to the absence of said compound identifies said compound as an enhancer of said phosphorylation.

The invention in its various aspects may include and/or involve among others one or more of the following: (1) analytes, (2) tracers, (3) specific binding partners, and (4) modulators. In most cases, the format of the assay will place constraints on the preferred relative concentrations of these components and on the preferred lifetime of the luminophore associated with the tracer. For example, in a competition assay, the concentration of binding partner is preferably at least about equal to the concentration of tracer (to permit stoichiometrically an appreciable fraction of the tracer to be bound), and the affinity of the binding partner for the tracer is preferably such that the dissociation coefficient $K_d$ describing interaction between the binding partner and tracer is about equal to the concentration of the free binding partner (so that the fraction of bound tracer is not too close either to 0 or 1, to maximize sensitivity). Moreover, the lifetime of the luminophore associated with the tracer preferably is longer than the rotational correlation time of free tracer and shorter than the rotational correlation time of bound tracer, and, all else being equal, ideally equal to the geometric mean of the two rotational correlation times of free and bound tracer (to obtain the largest polarization change upon binding).

The "modified substrate" may be a posttranslationally modified protein or peptide, such as a phosphorylated peptide, and the sample may be an intact sample or a set of fragments thereof. The substance for which the tracer and modified substrate compete is typically an antibody, but includes any specifically binding substance that results in a change in the luminescence polarization of the tracer.

The invention also relates to luminescent tracers used in specific embodiments of the invention, as well as cell lines that provide intracellular specific binding pair members useful in the assays.

The method of the invention takes advantage of the change in luminescence polarization of a luminescently labeled substance when the substance becomes bound to the opposing member of a specific binding pair of which the substance itself is a member. Typically, the substance is a peptide or protein.

By "opposing member of a specific binding pair" is meant a member or a partner of the pair that binds specifically but noncovalently to a subject material that is the other members or partners. Such terminology is familiar in a number of contexts. Thus, antigens and antibodies are opposing members of specific binding pairs, as are ligands and receptors, and as are avidin and biotin. Depending on the application, a specific binding partner may bind substantially exclusively only to a particular species, such as a phosphorylated peptide having a particular sequence as opposed to other sequences, or only to a particular class of species, such as phosphorylated peptide as opposed to an unphosphorylated peptide.

To function in a competition assay, both competitors must bind substantially to the opposing member of the specific binding pair. Thus, the competitors may be the analyte to be assayed and the analyte in luminescently labeled form, i.e., coupled to a luminescent material. Peptides are convenient analytes, as labeled forms can be prepared easily. Thus, the analyte itself is typically a peptide and especially a modified form of the peptide that can be distinguished through specific binding from the unmodified form. Thus, as used herein, "modified substrate" refers to a substrate for an enzyme-catalyzed reaction that has been converted to product. Exemplified herein is an assay where the modified substrate is the phosphorylated form of an unmodified peptide, wherein antibodies can be prepared which bind specifically to the phosphorylated form.

In the illustrated embodiment, the tyrosine residue (Y) in the peptide is phosphorylated. However, other modifications to peptides and proteins are well known and can be detected by using such modified substrates as analytes as well. A summary of known posttranslational modifications of proteins and peptides is set forth in Advances in Enzymology (1993) 67:265-299. For example, the N-terminus of various peptides can be modified by acylation (e.g., by formyl, acetyl, myristoyl, lauroyl, tetradeca (mono and di) enoyl, $\alpha$-ketoacyl, and aminoacyl groups), and also can be derivatized with methyl, pyrrolidone carboxyl, gluconuryl, and glycosyl groups. Similar substitutions can take place at the nitrogen of lysine, including lipoyl substitution, biotinyl, ubiquitinyl, phosphoryl, pyrridoxyl, and glycosyl. Hydroxy groups also can be added to the lysine at the $\delta$ position, which can then be further derivatized. Arginine can be derivatized as the ADP-ribose derivative, alkyl derivative, and additional substitutions. A variety of acyl groups, specifically of fatty acids, can be reacted with the sulfhydryl group of cysteine, along with coenzymes, prenylation, and the like. Tyrosine can be substituted at the hydroxyl group by a variety of substituents and can be halogenated as well. Modifications may be a result of enzymatic or nonenzymatic processes, for example, glycosylation of hemoglobin in diabetic patients.

Common groups that are phosphorylated include tyrosine, serine, and threonine.

In the foregoing cases, antibodies can be made to the modified proteins that fail to react significantly to either the unmodified or modified form. Such antibodies are useful as the opposing members of the specific binding partner for analyte. Thus, antibodies can be prepared by using the modified substrate as an immunogen, harvesting appropriate antibody producing cells and immortalizing them, and performing appropriate screening tests. The tests select immortalized cells that produce antibodies that bind to the modified substrate, but discard those that also bind to the substrate itself. In this manner, a suitable opposing member of a specific binding pair can, in principle, be prepared with respect to any modified or unmodified substrate.

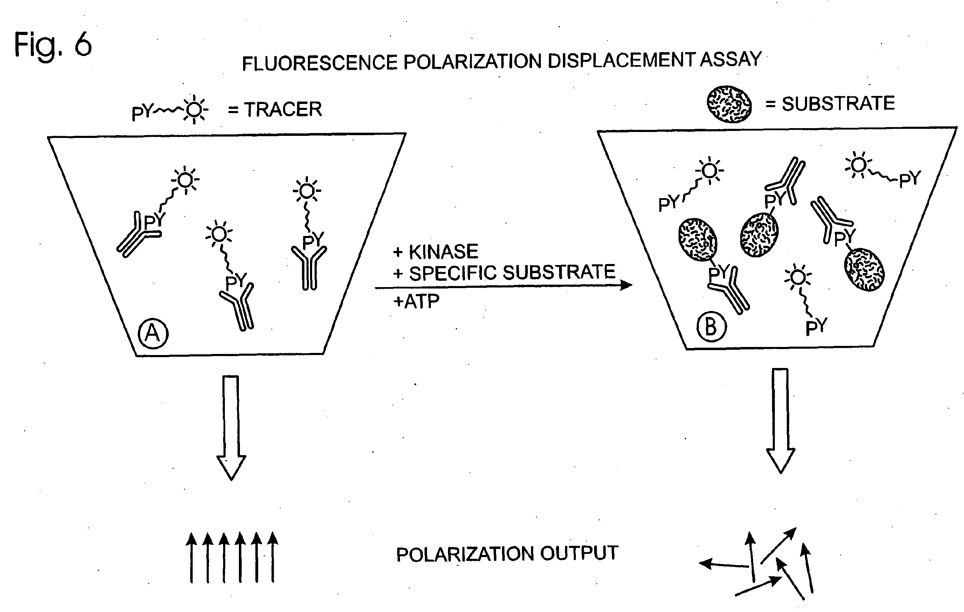

Figure 6 shows a diagrammatic representation of the overall aspects of the assay. Antibodies to phosphoryl tyrosine (pY) are illustrated as the opposing member of the specific binding pair. As shown in flask A, a significant fraction of the luminescent tracer is bound to the specific antibodies, and the luminescence polarization output is high. In flask B, however, a kinase that phosphorylates the tyrosine residues on a substrate is introduced, and the modified substrate is thus present in flask B. The modified substrate displaces the luminescent tracer from the antibodies and thus reduces the polarization of the sample.

In its simplest form, the assay can be constructed so as simply to measure the concentration of the analyte. The analyte can be any substance for which a luminescent tracer can be prepared by coupling a luminescent label to it or a close analog, and for which an opposing member of a specific binding pair can be obtained which is competitively bound by the tracer and the analyte. Thus, any arbitrary substance can be used as the analyte in this assay. However, the assay of the invention is particularly useful when the analyte is a "modified substrate" that is produced by an enzyme-catalyzed reaction converting a substrate to its modified form. Thus, if the analyte is a modified substrate formed through a catalyzed reaction, the assay may be designed so as to measure the activity of the catalyst and to screen for compounds that enhance or inhibit this activity. If the activity is associated with a receptor, agonists and antagonists for the receptor may be measured by the assay of the invention.

Thus, by measuring the concentration of analyte as a function of time, or at a suitable preset time, the level of catalytic activity in its formation can be assessed. The effect on the time course of analyte concentration as produced by additional substances serves to indicate whether these substances are enhancers or inhibitors of this activity.

The luminescent label can be any of a number of dyes as long as the dye exhibits luminescence polarization when the substance to which it is conjugated is bound to the opposing member of the specific binding pair. Fluorescein is a typical fluorescing unit, which can be coupled, for example, through the corresponding isothiocyanate to a free amino group in the tracer substrate. Other luminescent compounds that are suitable include dansyl, rhodamine, and Texas Red.

To conduct the assay, a sample of the modified substrate or an analog representing it is first coupled to a luminescent label through standard linkage technology known in the art. The resulting labeled tracer is then titrated with the opposing member of its specific binding pair, which also is capable of binding to the modified substrate per se. The substrate for an enzyme catalyzed reaction, the enzyme, and any necessary cofactors are combined to form a catalysis mixture, which is added immediately or at fixed intervals to buffer containing the specific binding pair member and the luminescent tracer. As the enzyme catalyzes the conversion of the substrate to the modified form, the modified form is detected through competition with the luminescent tracer. The amounts of reagents are adjusted so as to provide distinction from background polarization and to obtain meaningful results. The amount of modified substrate under a variety of conditions of concentration of components or at various times is then measured by determining luminescence polarization. Measurement of luminescence polarization is well known and can be conducted with standard laboratory equipment.

If the bound form of the tracer has a higher luminescence polarization than the tracer in free form, the luminescence polarization of the sample will decrease as more modified substrate is formed. By measuring the luminescence polarization at various times, then, the rate of conversion of substrate to modified substrate can be determined directly. Alternatively, the level of enzyme can be measured by determining the luminescence polarization at a set time at a series of dilutions of the catalysis mixture containing the enzyme, substrate, and cofactors. Accordingly, the level of enzyme that converts substrate to modified substrate can be determined, and the effect of any stimulator or inhibitor molecules on the activity of the enzyme can be assessed.

The following examples are intended to illustrate but not to limit the invention. They illustrate the application of the invention method to study the features of the insulin receptor, to determine the kinase activity of the receptor, and more specifically of the cytoplasmic kinase only domain, and thereby to identify agonists and antagonists of the receptor. The insulin receptor contains two $\alpha$ chains and two $\beta$ chains which are assembled at the cell membrane. The $\alpha$ chains are exposed on the outside of the cell and contain the insulin-binding site. The $\beta$ chains extend to the interior of the cell and contain a binding site for ATP, as well as tyrosine residues that are phosphorylated when the receptor is stimulated by insulin. Thus, the peptides not phosphorylated at the relevant tyrosine residues are the substrate for the autophosphorylation activity of the insulin receptor, and the pY form is the modified substrate.

A primary substrate for the kinase activity of the activated receptor, IRS-1, undergoes tyrosine phosphorylation as an early step in the insulin receptor signaling pathway, and it too could be assayed by the method of the invention.

Further, sites of phosphorylation within a protein can be mapped following digestion of the protein and HPLC separation of the fragments. Such a method could map a protein for the location of modification sites, and would include as first and second steps the digestion of a protein with a protease to obtain fragments and then the separation of the fragments. The fragments are then treated with a luminescence polarization mixture comprising a tracer and an opposing specific binding pair member, where the tracer and any modified fragments compete with one another for binding to the opposing specific binding pair member. A detection of a change in luminescence polarization would indicate a modified fragment.

The following examples are intended to illustrate without limitation various aspects of the invention.

Example 1

Effect of Binding on Luminescence Polarization

As the ultimate intention is to measure by pY content the modified substrate of the insulin receptor kinase, a phosphorylated peptide that mimics the portion of the substrate that will become phosphorylated by virtue of the kinase activity is used as the labeled tracer. This peptide may be relatively simple, and thus applicable to many kinases, or it may be a more specific mimic of one particular kinase substrate. The phosphorylated form of the mimicking peptide will thus bind to anti-pY antibodies of greater or lesser specificity as will the modified substrate itself. To determine whether such a tracer would indeed exhibit enhanced luminescence polarization when bound to anti-pY antibodies, the following preliminary studies were conducted.

The peptide to be coupled to luminescent label was of the formula GEEGYMPMGK, where the tyrosine residue is chemically phosphorylated, and the C-terminal lysine is derivatized on the $\epsilon$ amino group with a biotin. The derivatization with biotin is irrelevant to the claimed assay. The tracer form of this peptide was prepared by coupling fluorescein to the amino terminus by reaction with fluorescein isothiocyanate (FITC).

The luminescence polarization experiment was tested on two types of readers: an FPM-1 Fluorescence Polarization Analyzer, a single $12 \times 75$ mm tube reader (Jolley Consulting and Research, Inc.), and a FPM-2, a 96-well reader

The single-tube reader is a more sensitive instrument, and can use less tracer. In the FPM-1, the tracer form of the peptide (GEEGYMPMGK) coupled with fluorescein to the amino terminus was used at 0.3 nM. To transfer this technology to multiple samples, the assay was transferred to a 96-well format. In doing so, the sensitivity of the instrument is less, and as a result more tracer is required to give adequate signal. We were able to see 7.5 and 15 nM of the tracer form of the peptide coupled with fluorescein. For the simpler tracer, FL-phosphotyramine, we had to use 35 nM of the tracer.

Phosphotyramine has been conjugated to fluorescein to give the tracer FL-phosphotyramine. When mixed with an anti-phosphotyrosine antibody, it can be competed off with N-Acetyl-O-Phosphotyramine. N-Acetyl-O-Phosphotyramine is used as the calibrator for the assay in this instance.

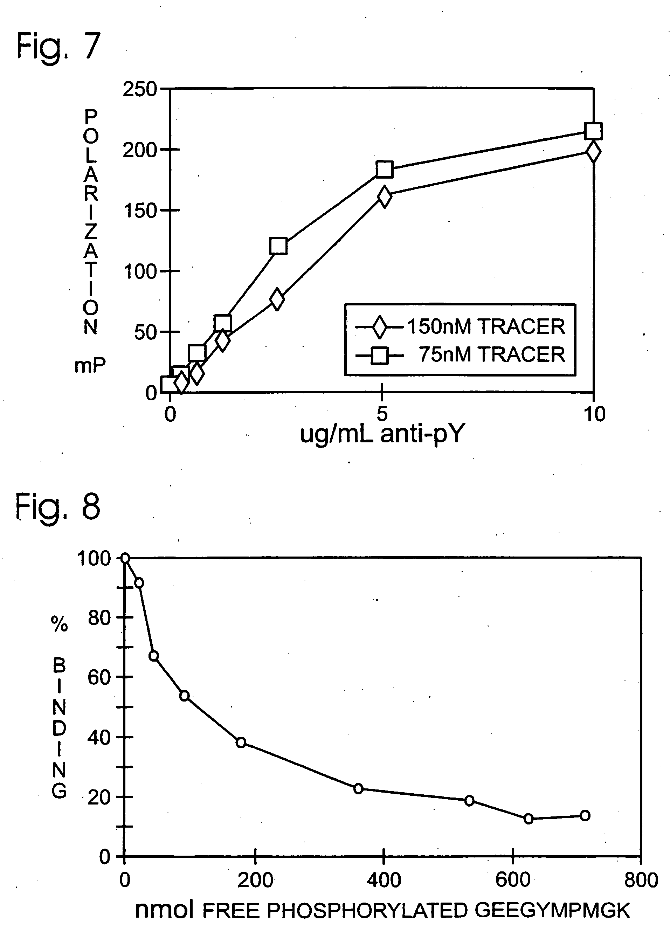

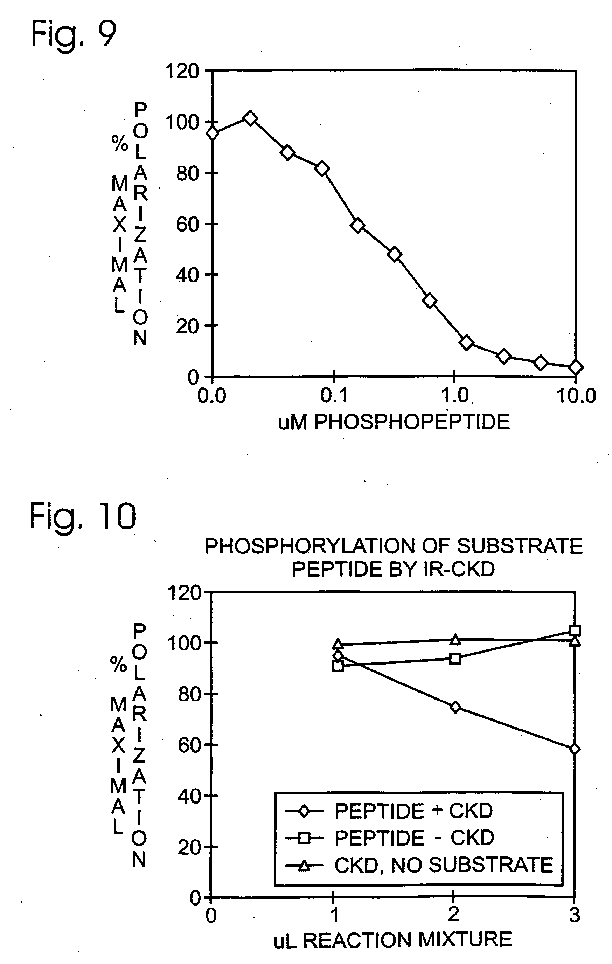

It also is necessary, of course, that the labeled tracer is effectively displaced when an unlabeled form of the phosphorylated peptide is added. Upon addition of 2 $\mu$g/mL of the unlabeled phosphorylated peptide, the polarization diminished to less than 10% of control. The competition of labeled tracer and the unlabeled peptide as a function of the concentration of the unlabeled peptide is further shown in Figures 8 and 9. In Figure 8, 1 nM tracer and 0.3 $\mu$/mL anti-pY antibody are incubated in 150 mM NaCl, 50 mM Hepes, pH 7.6, and 0.01% bovine IgG in the presence of various amounts of unlabeled phosphorylated peptide. As shown, over the range of 0-700 nM, the unlabeled peptide competes as a function of concentration with the tracer. (Here, "tracer" refers to the phosphorylated peptide set forth above carrying the luminescent label, and "unlabeled peptide" refers to the same peptide without the fluorescein conjugate.)

Figure 9 shows the competition curve in the presence of 75 nM tracer and 5 $\mu$g/mL anti-pY antibody as a function of unlabeled peptide concentration. This competition curve is indicative of the concentration dependence on unlabeled competitor over the range of 0-1 $\mu$M.

Example 2

Measurement of Kinase Activity

Several peptides were used as substrates for phosphorylation. These peptides were:

| KS2: | biotin-EGPWLEEEEEAYGWMDF-amide (Boehringer-Manheim Catalog No. 1768719); |
| D-peptide: | DYMTMQIG; |
| S-peptide: | SRGDYMTMQIG. |

Each of these substrates at about 10 $\mu$M was incubated with or without 200 ng of insulin receptor cytoplasmic kinase domain (IR-CKD) in 100 $\mu$L assay buffer (15 mM Tris, pH 7.5, 0.05% BSA, 2 mM MnCl$_2$, 10 mM MgCl$_2$, 200 $\mu$M ATP) for one hour and then stopped by bringing the solution to 20 mM EDTA. This is the "catalysis mixture."

Aliquots ranging from 10-50 $\mu$L of the catalysis mixture were added to tubes containing 0.8 mL luminescence polarization (FP) buffer (50 mM Hepes, pH 7.6, 150 mM NaCl, 0.01% bovine IgG, 1 mM sodium vanadate, 20 mM EDTA) containing 1.0 nM of the tracer of Example 1. A 0.3 $\mu$g portion of anti-pY (4G10) was added to each tube. The tubes were vortexed and incubated for 15 minutes in the dark at room temperature. Using a standard calibration curve, the amount of modified substrate was determined. As shown in Table 1, KS2 was a better substrate than either of the D or S peptide essentially by a factor of 10.

| Table 1 nMol pY Created in Original Reaction Volume | | | | |
|---|---|---|---|---|
| Tube No. | S-peptide | D-peptide | KS2-peptide | KS2 but no CKD |
| 1 | 569 | 639 | 5411 | 66 |
| 2 | 727 | 625 | 5772 | 74 |
| 3 | | 593 | 6412 | |
| 4 | | | 6046 | |
| 5 | | | 6452 | |
| PmoL pY/min/$\mu$g CKD | 4.9 | 4.7 | 46.7 | <0.7 |

The results obtained in the foregoing assay were evaluated when various amounts of the catalysis mixture were added to the tubes for the luminescence polarization competition reaction. The results are shown in Figure 10, which is mainly a showing of the signal as proportional to the concentration of CKD. As expected, if the reaction mixture did not contain CKD or did not contain substrate, no reduction of polarization was seen. However, if both CKD and substrate were present, the level of reduction of polarization decreased with the amount of catalysis mixture added.

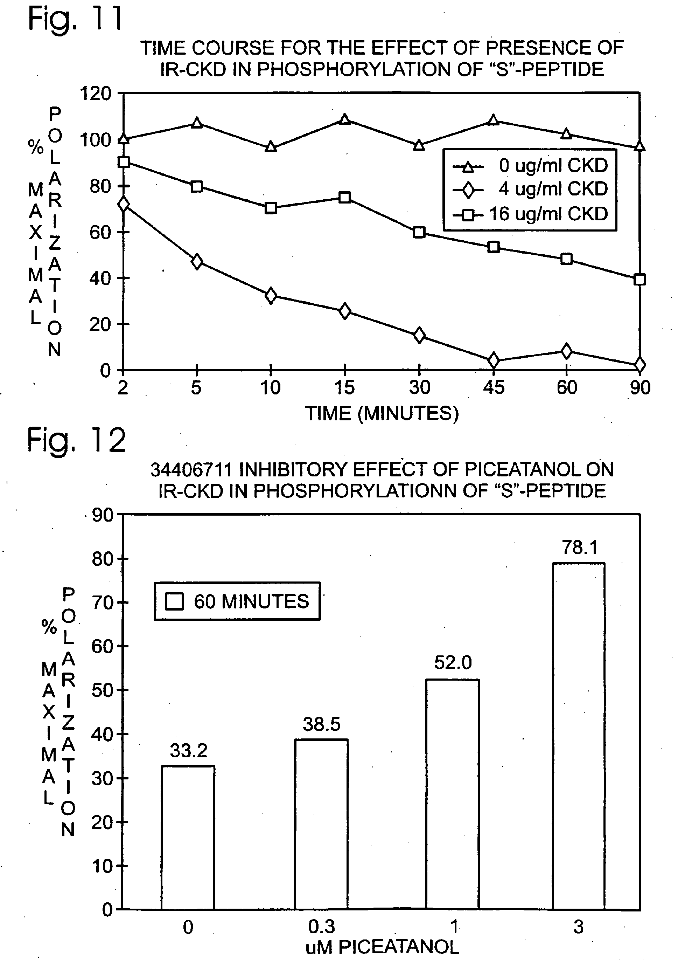

To measure kinase activity quantitatively, rather than simply the necessity for kinase and its substrate, the foregoing experiments were repeated but sampled at various times and using various CKD concentrations. The results are shown in Figure 11, which is mainly a showing of the signal at specific concentrations of CKD as proportional to time. As shown, higher concentrations of CKD enhanced the rate of diminution of luminescence polarization in the reaction mixture.

Example 3

Effect of Agonists and Antagonists

The foregoing experiment, in which luminescence polarization was measured as a function of time, was repeated with CKD at 4 μg/mL concentration in the presence of a known inhibitor, piceatannol, as measured at a fixed time using 5 μL of the catalysis mixture. The results are shown in Figure 12, indicating that the rate of diminution of luminescence polarization is diminished in the presence of the inhibitor.

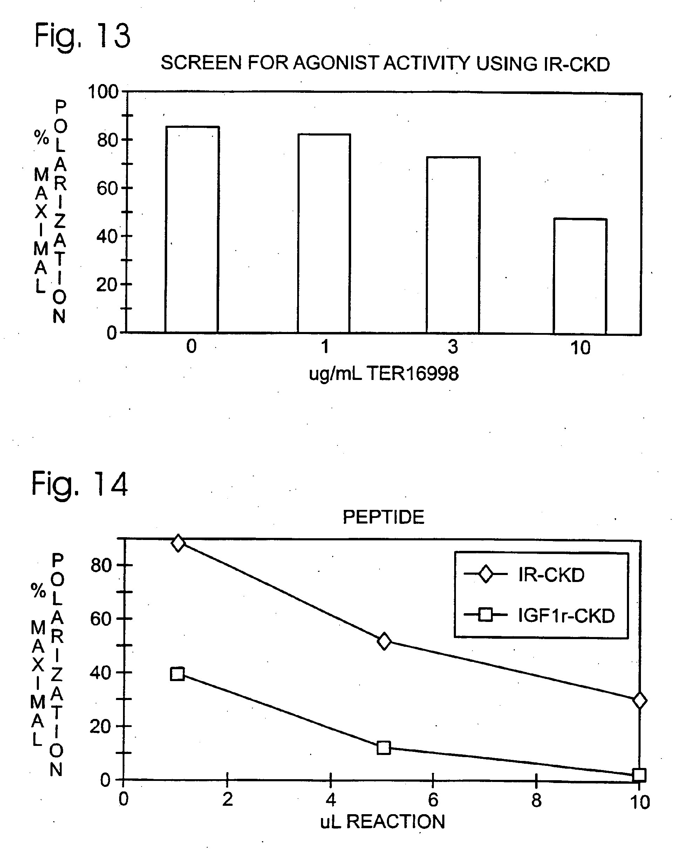

The opposite result in the presence of a known agonist of the insulin receptor is shown in Figure 13. As indicated, higher concentrations of TER 16998, known to stimulate the insulin receptor, at increasing concentrations provided significant decreases in maximal polarization when this determination was run as an end-point assay using 5 μL of the catalysis mixture.

A similar assay can be used to determine kinase activity of alternative receptors, such as the IGFIr receptor as shown in Figure 14. The results in this figure show that the decrease in maximal polarization can be used as a measure of either IR-CKD activity or IGFIr-CKD activity.

Example 4

Peptide Mapping

Phosphorylated IR-CKD (83 μg) was hydrolyzed with endoproteinase Lys-C lysyl endopeptidase protease (EC. 3.4.21.50), (Wako Chemical USA, Inc., Richmond, VA), and the reaction mixture subjected to High Pressure Liquid Chromatography (HPLC) using a Phenomenex (Torrance, CA) Jupiter 5 micron C 18 300 Å 250 × 2.0 mm reversed phase column, with detection at 214 nm. The samples were eluted using a continuous elution gradient beginning with 95% Buffer A-5% Buffer B to 5% Buffer A-95% Buffer B. The composition of Buffer A was: 5% acetonitrile; 0.1% trifluoroacetic acid (TFA); 95% water. The composition of Buffer B was: 95% acetonitrile; 0.085% trifluoroacetic acid (TFA); 5% water.

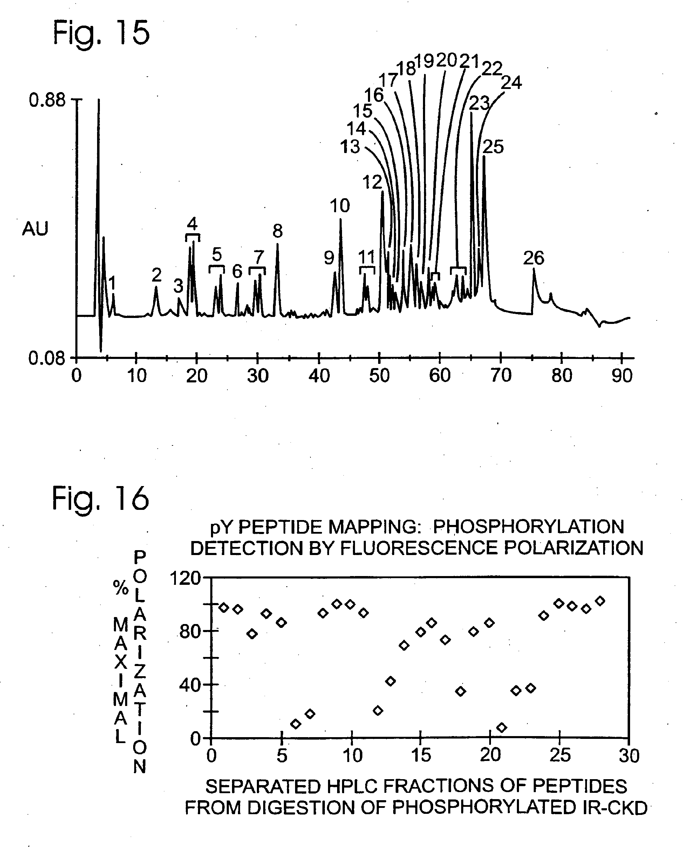

The fractions representing individual peptide peaks seen in Figure 15 were assayed as described in Example 3 for the presence of phosphorylated tyrosine in the fragment. As shown in Figure 16, fractions 6, 7, 12, 13, 18, 21, 22, and 23 contain phosphorylated peptides.

Example 5

Serine/threonine (S/T) Kinase Assays

The invention provides apparatus, methods, compositions, and kits for determining the presence and activity of S/T kinases. The methods may include homogeneous luminescence assays, so that difficulties and complexities associated with heterogeneous assays and/or radioactivity may be avoided. The methods, compositions, and kits may include one or more generic kinase substrates, and binding partners that interact with that substrate and/or phosphorylated forms of that substrate. The invention is particularly well suited to screening for modulators of S/T kinase activity, as in drug discovery applications, although the invention also may be used for other applications.

Methods provided by the invention generally include the following steps:

(1) providing a protein substrate having one or more sites capable of being phosphorylated by an S/T kinase;

(2) providing a phosphate donor capable of donating a phosphate group to the protein substrate in the presence of an S/T kinase;

(3) providing a specific binding partner capable of specifically binding the phosphorylated protein substrate, but generally incapable of specifically binding the unphosphorylated protein substrate, or vice versa;

(4) contacting a sample with the protein substrate, phosphate donor, and specific binding partner, so that the protein substrate is phosphorylated if the sample includes S/T kinase activity; and

(5) detecting S/T kinase activity by detecting changes in interactions between the protein substrate and the specific binding partner.

The protein substrate generally comprises any amino acid, peptide, or protein, or fragment, derivative, or analog thereof, capable of being phosphorylated by an S/T kinase. Such amino acids, peptides, proteins, or fragments, derivatives, or analogs thereof, preferentially will include one or more serine and/or threonine residues.

The protein substrate may include a generic substrate capable of being phosphorylated by a variety of S/T kinases. Preferred generic substrates may include histone type III-ss, human/bovine myelin basic protein (MBP), and fragments of MBP including all or part of amino acids 4-14. The lattermost substrate has the following sequence:

EKRPSQRSKYL

Here (and throughout the application), S is the one-letter abbreviation for serine, T is the one-letter abbreviation for threonine, and E, K, R, ... are the one-letter abbreviations for other amino acids, as specified in the Appendix. Here, S and T are underlined so that the location(s) of potential phosphorylation sites may be more easily identified. Preferred substrates also may include serine and/or threonine amino acids followed in the C-terminal direction by a proline residue, particularly immediately after (i.e., within one amino acid) of the serine or threonine. Preferred substrates also may include amino acid sequences having one or more serine and/or threonine amino acids, enriched for several amino acids to one or both sides of at least one serine and/or threonine by basic amino acids, such as lysine, arginine, and histidine, particularly within about three amino acids in the C-terminal direction. Basic amino acids, including lysine, arginine, and histidine, generally are positively charged under physiological conditions. These substrates are preferred because they may have enhanced recognition by kinase enzymes.

Preferred substrates generally are short, so that they may undergo a more significant change in size and hence rotational correlation time upon binding to a binding partner (such as an antibody). Here, short generally means shorter than about 40 amino acids, and especially shorter than about 15 amino acids.

The phosphate donor generally comprises any group capable of donating a phosphate group to the protein substrate in the presence of an S/T kinase. A preferred phosphate donor is adenosine triphosphate, or ATP.

The specific binding partner generally comprises any compound capable of specifically binding the phosphorylated protein substrate, but incapable of specifically binding the unphosphorylated protein substrate, or vice versa. Specific binding means binding to the specific binding partner to the exclusion of binding to most other moieties. Specific binding can be characterized by a binding coefficient. Generally, specific binding coefficients range from $10^{-4}$ M to $10^{-12}$ M and lower, and preferred specific binding coefficients range from $10^{-9}$ M to $10^{-12}$ M and lower.

In some assays, preferred specific binding partners, or fragments, derivatives, or analogs thereof, may be coupled to solid supports or other moieties, including beads and walls. In other assays, fragments, derivatives, or analogs of preferred specific binding partners may be used, if such fragments, derivatives, and analogs retain their specificity and binding affinity for their binding partners.

Preferred specific binding partners include immunological specific binding partners, such as antibodies. In particular, preferred specific binding partners include antibodies against unphosphorylated or phosphorylated forms of the preferred protein substrates listed above, including antibodies against the following phosphorylated MBP fragments:

EKRPpSRSKYL
EKRPSQRpSYL
EKRPpSRpSYL

Here, a "p" is used to denote phosphorylation of the subsequent amino acid. Preferred specific binding partners also include antibodies against the following phosphorylated peptides:

| | |
|---|---|
| KRREILSRRPpSYRK | FpTPLQ |
| KHFPQFpSYSAS | RKRpTLRRL |
| pSPELERLIIQC | LRRApSLG |
| GSPSVRCSpSMpS | KKLNRTLpSVASL |
| RSRHSpSYPAGT | RPRAApTF-NH$_2$ |
| LpTPLK | LRRApSLG-NH$_2$ |

The lattermost two peptides are amidated at the C-terminal, which may act to stabilize the peptides.

These or other protein substrates, or fragments, analogs, and/or derivatives of such substrates, or cells expressing such substrates, fragments, analogs, and/or derivatives can be used as immunogens to produce immunological binding partners thereto. Generally, preferred immunogens are phosphorylated forms of preferred substrates, especially substrates in which phosphorylated serines and/or threonines are flanked by basic amino acids, because serine, threonine, and phosphate groups alone may be too generic to induce good immune responses. Immunological binding partners include polyclonal and monoclonal antibodies. Immunological binding partners also include chimeric, single chain, and humanized antibodies, as well as Fab fragments and the products of Fab expression libraries.

Immunological binding partners, including antibodies and fragments, analogs, and/or derivatives of such antibodies, can be produced by various generally known procedures. For example, antibodies against protein substrates can be obtained by injecting or otherwise administering the protein substrates into an animal, where the animal preferably is different in species than the animal from which the protein substrate is derived. The antibody so obtained will then bind the protein substrates. In this manner, even a sequence encoding only a fragment of a protein substrate can be used to generate antibodies that will bind the whole protein substrate.

Monoclonal antibodies can be prepared by any technique that provides antibodies produced by continuous cell line cultures. Examples include the hybridoma technique (Köhler and Milstein, Nature 256:495 (1975)), the trioma technique, the human B-cell hybridoma technique (Kozbor et al., Immunology Today 4:72 (1983)), and the EBV-hybridoma technique to produce human monoclonal antibodies (Cole et al., in Monoclonal Antibodies and Cancer Therapy, Alan R. Liss, p. 77 (1985)).

Single-chain antibodies can be prepared using any technique for producing single-chain antibodies. Examples include the method described in U.S. Patent No. 4,946,778.

The sample generally comprises any composition for which the presence or activity of S/T kinases is to be tested. The sample may be natural, artificial, or a combination thereof. The sample may include proven or putative S/T kinases, including cyclic AMP-dependent kinase (A-kinase), cyclic GMP-dependent kinase (G-kinase), protein kinase C (C-kinase), $Ca^{2+}$-calmodulin-dependent kinase (CaM-kinase), phosphorylase kinase, MAP-kinase, and TGF-β receptor, among others. The sample also may include proven or putative modulators of S/T kinases, including inhibitors and/or enhancers of S/T kinases.

The sample may be contacted with the protein substrate, phosphate donor, and specific binding partner using any method for effectuating such contact. A preferred method is by mixing the materials in solution, although other methods such as attaching one or more components to a bead or surface also may be used.

S/T kinase activity within the sample may be detected using any method for detecting specific binding between the unphosphorylated or phosphorylated protein substrate and the specific binding partner. A preferred method is a luminescence assay, especially a homogeneous luminescence polarization assay.

Luminescence S/T kinase assays are constructed so that interaction of the protein substrate and specific binding partner may be correlated with some change in luminescence. In particular, luminescence assays are constructed to detect changes in the interaction between the protein substrate and specific binding partner brought about by phosphorylation of the protein substrate by S/T kinases, and to qualitatively or quantitatively correlate such changes with such activity. Suitable assays may measure intrinsic or extrinsic properties of the luminescence, and may include noncompetitive, competitive, and sandwich assays.

Luminescence assays may be performed by collecting luminescence light from luminophores associated with the protein substrate, specific binding partner, and/or other moieties whose luminescence reflects interactions of the protein substrate and specific binding partner. Such luminophores may be intrinsic to the protein substrate, specific binding partner, and/or other moieties, such as tryptophan, tyrosine, or luminescent gene products such as green fluorescent protein. Such luminophores also may be extrinsic, as described below. Extrinsic luminophores may be associated with the protein substrate, specific binding partner, and/or other moieties covalently or noncovalently.

The compositions and kits provided by the invention include but are not limited to compositions and kits for practicing the methods described above. For example, compositions and kits may include protein substrates, specific binding partners, and fragments, analogs, and derivatives thereof. Compositions and kits also may include reactive and luminescently labeled versions of such protein substrates, specific binding partners, fragments, analogs, and derivatives.

The methods, compositions, and kits provided by the invention may be practiced using apparatus, methods, and compositions described in the above-identified patent applications. For example, luminescence may be detected using high-sensitivity luminescence apparatus, including those described in U.S. Patent 6,071,748, filed April 17, 1998 and PCT Patent Application Serial No. PCT/US98/23095, filed October 30, 1998 (Publication No. WO/1999/023466). Luminescence also may be detected using high-sensitivity luminescence methods, including those described in PCT Patent Application Serial No. PCT/US99/01656, filed January 25, 1999 (Publication No. WO/1999/037203), and PCT Application Serial No. PCT/US99/03678, filed February 19, 1999 (Publication No. WO/1999/042817). Luminescence also may be detected using sample holders optimized for performance with the above-identified high-sensitivity luminescence apparatus and methods, including those described in PCT Patent Application Serial No. PCT/US99/08410, filed April 16, 1999 (Publication No. WO/1999/054711).

Example 6

Tyrosine Kinase Assays

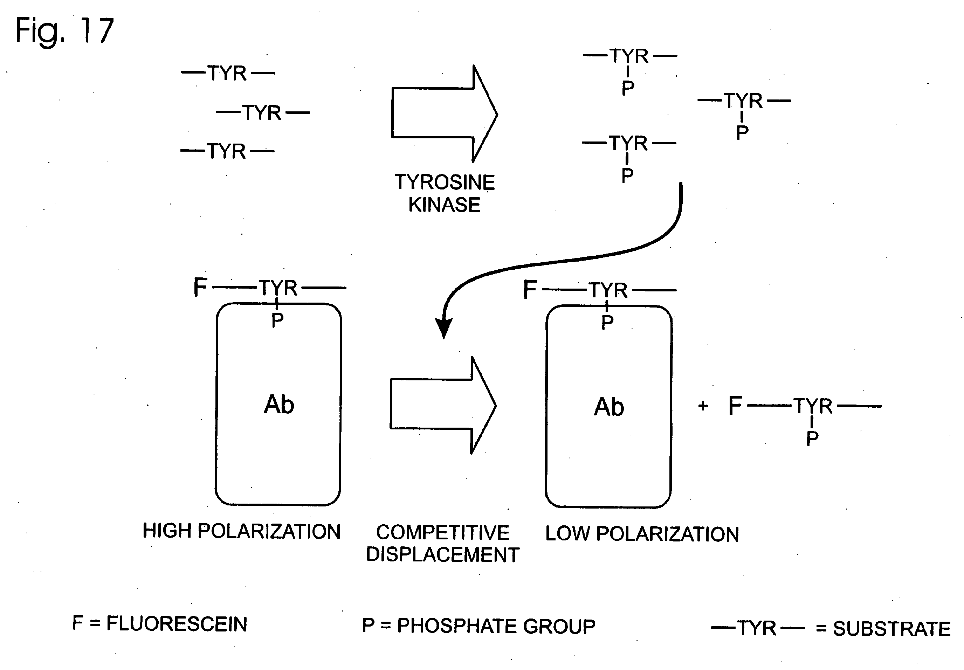

TK enzymes function by phosphorylating tyrosine residues on substrate proteins or peptides. The amount of phosphorylated protein in a solution containing TK enzymes thus may be used as an assay of TK enzyme activity. TK activity may be quantified by a competitive assay. In a competitive TK assay, as shown schematically in Figure 17, proteins phosphorylated by TK enzymes ("enzyme product") compete with a luminescently labeled phosphotyrosine analog ("tracer") for binding sites on an anti-phosphotyrosine antibody ("antibody"). The tracer has low luminescence polarization when free and high luminescence polarization when bound to antibody. Hence, luminescence polarization will be low when TK enzyme activity is high, because enzyme product will be present to compete tracer off the antibody. Conversely, luminescence polarization will be high when TK enzyme activity is low, because enzyme product will not be present to compete tracer off the antibody.

A known TK assay includes six steps, as follows:

1) Run kinase reaction in a microplate
2) Stop kinase reaction with chelating EDTA solution
3) Pipette an aliquot of the stopped reaction mixture into a second microplate
4) Add antibody to second microplate
5) Add tracer to second microplate
6) Measure luminescence polarization in second microplate

Although this six-step assay is adequate to measure TK enzyme activity, it has a number of shortcomings. The assay involves a relatively large number of steps, increasing the likelihood of error. Moreover, the assay requires that sample be transferred between different containers, increasing waste and cost and further increasing the likelihood of error. These shortcomings are particularly significant if the assay is used to screen new drugs, because such screening typically involves conducting massive numbers of assays, frequently hundreds of thousands. Under such conditions, throughput is significantly decreased by having to conduct six steps for each assay, and waste is significantly increased by having to use two sample containers for each assay.

The invention may address these and other shortcomings by providing assays for TK enzyme activity that require fewer than six steps and that involve only a single sample container.

In a first embodiment, the invention includes a TK assay involving only five steps and only a single container. This assay includes the following steps:

1) Run kinase reaction
2) Add a stop solution to stop kinase reaction
3) Add antibody
4) Add tracer
5) Measure luminescence polarization

In a second embodiment, the invention includes a TK assay involving only four steps and only a single container. This assay includes the following steps:

1) Run kinase reaction
2) Add a stop solution to stop kinase reaction
3) Add a mixture of antibody and tracer
4) Measure luminescence polarization

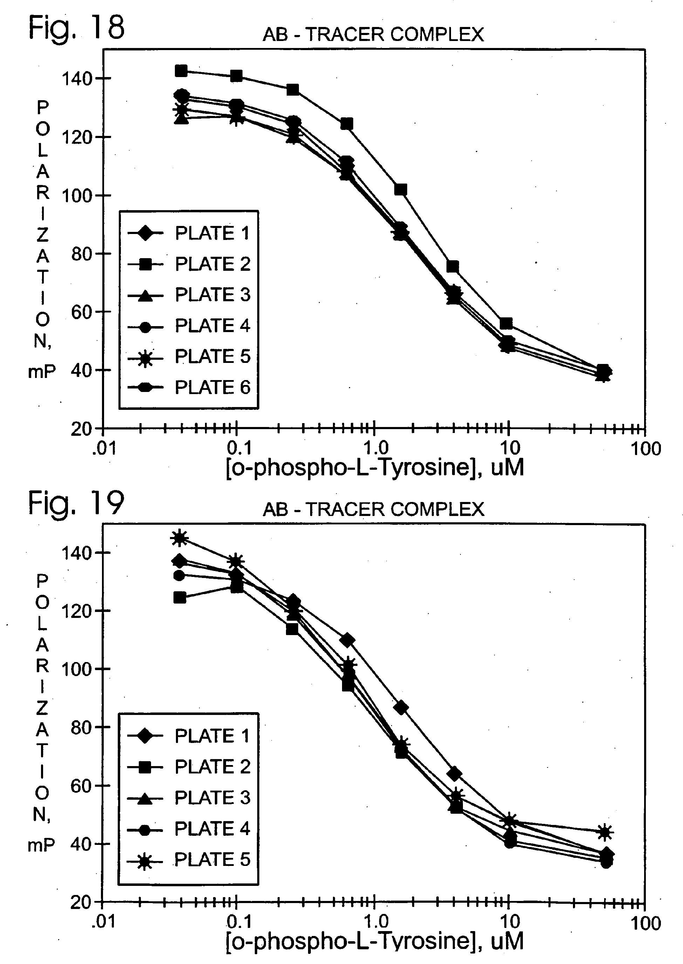

Figure 18 shows results generated with the second protocol. Competitive curves demonstrate assay performance in which a mixture of tracer and antibody is added to the simulated enzyme product (o-phospho-L-tyrosine). The tracer and antibody were incubated together in the mixture for 30 minutes to equilibrate before addition to the phosphotyrosine in the assay plate. Plates 2, 3, 5, and 6 correspond to this method, with the same final antibody/tracer solution prepared by different intermediate dilutions. Plates 1 and 4 were run according to the first simplification. Plates 1-3 and 4-6 were run on two separate days.

In a third embodiment, the invention includes a TK assay involving only three steps and only a single container. This assay includes the following steps:

1) Run kinase reaction

2) Add a mixture of stop solution to stop kinase reaction, antibody, and tracer

3) Measure luminescence polarization

Figure 19 shows the results generated with the third protocol. Competitive curves demonstrate assay performance in which a mixture of EDTA, tracer, and antibody is added to the simulated enzyme product (o-phospho-L-tyrosine). The EDTA, tracer, and antibody were incubated together in the mixture for 30 minutes to equilibrate before addition to the phosphotyrosine in the assay plate. In plates 2 and 3 the concentration of EDTA was 20 mM; in plates 4 and 5 it was 50 mM. In plates 2 and 4, the phosphotyrosine solution to which the mixture was added had no divalent cations (necessary for kinase activity). In plates 3 and 5 the phosphotyrosine plates did contain divalent cations, more fully simulating actual assay conditions. Plate 1 was a control, run according to the first simplification.

These methods should reduce significantly the time, waste, and cost associated with conducting TK assays, with no significant loss in biochemical performance. In each embodiment, the step to run the kinase reaction may include running the reaction in a microplate. In addition, the step to add a stop solution may include adding a stop solution containing a chelator, such as EDTA.

These TK assays, particularly the second and third, yield surprisingly good results. The reason may depend in part on the relative time scales of the assay and on the kinetics of the equilibration of the tracer-antibody and product-antibody interactions. If the time scale of the equilibration is the slower, and if pre-formed tracer-antibody complex is added to product, the product may not fully displace the tracer before the luminescence-polarization is measured. If the antibody is added to the enzyme product, and then the tracer is added, equilibration may still be incomplete, but at least the enzyme product may block binding of tracer in a fashion directly related to its concentration. If the tracer and enzyme product are incubated together and then brought into contact with the antibody, equilibrium should be reached as long as the rate constants for antibody binding are similar for the tracer and enzyme product.

If the equilibration rate is relatively rapid, the order in which enzyme product, tracer, and antibody come into contact should be immaterial: the same equilibrium should be reached in all cases. It is, however, well known that antibody/antigen equilibration times can be long.

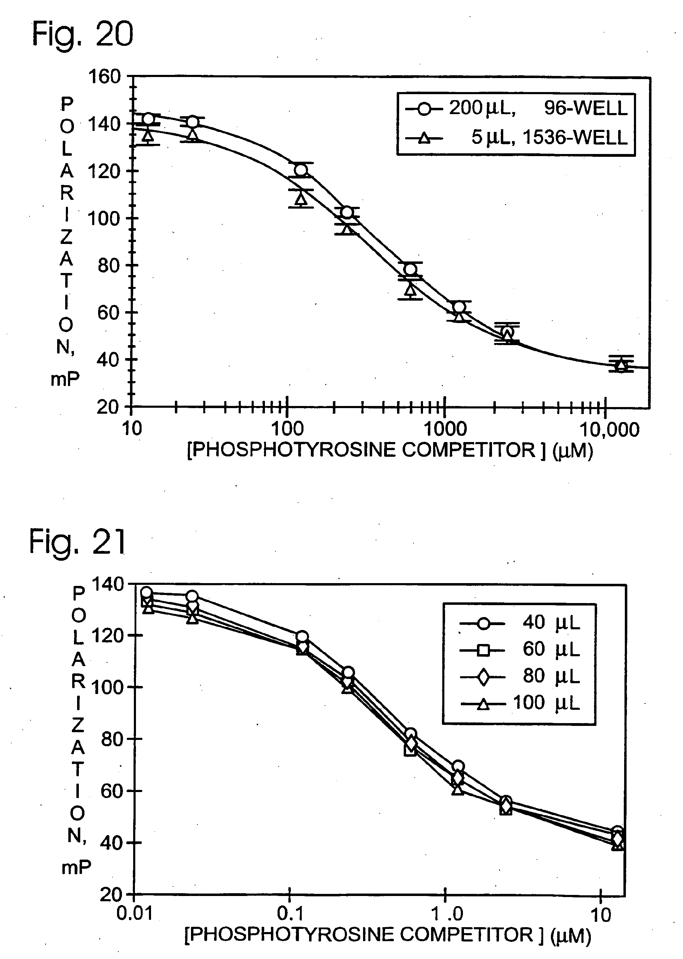

Figures 20-21 show results generated in additional studies of tyrosine kinase activity, showing how luminescence polarization may be used in a homogenous immunoassay format to screen for a modulator (inhibitor) of enzyme activity. Specifically, anti-phosphotyrosine antibody (1:750 dilution of ascites fluid) and fluorescein-labeled phosphotyramine (1 nM) were incubated with the indicated concentrations of phosphorylated tyrosine-kinase substrate (i.e., the enzyme product). The enzyme product competitively released the labeled compound from the antibody, decreasing the luminescence polarization, because the polarization of the label is high when bound to the antibody and low when displaced from the antibody by the enzyme product. Thus, the higher the enzyme activity, the lower the luminescence polarization.

Figures 20-21 show results obtained under a variety of assay conditions.

Figure 20 shows results for 200 $\mu$L samples in 96-well microplates and 5 $\mu$L samples in 1536-well microplates. Error bars, averaging 4 to 8 mP, are standard deviations. The phosphotyrosine $IC_{50}$ values determined from the data are 379 $\pm$ 22 nM for the 200 $\mu$L samples and 326 $\pm$ 30 nM for the 5 $\mu$L samples. Error bars are standard errors of the mean. Figure 21 shows results for 40, 60, 80, and 100 $\mu$L samples in 384-well microplates. Error bars, averaging 1 to 4 mP, are standard deviations. Error bars for polarizations in Figures 20-21 should be compared with the maximum change in polarization for the assay, which is about 110 mP.

Example 7

Phosphatase Assays

The invention also may be used to detect phosphatases rather than kinases if the protein substrate initially is phosphorylated and if the specific binding partner interacts with the substrate to detect dephosphorylation, for example, as shown in Figure 5.

Example 8

Peptide Tracers for Tyrosine Kinase Assays

In still another aspect, the invention is directed to specifically designed peptides that show enhanced affinity for binding to the 4G10 antibody, which is directed to phosphorylated tyrosine. Phosphorylated tyrosine is an indicator of an activated form of the insulin receptor and many other receptors such as EGF-receptor, and IGF-1 receptor, as well as nonreceptor tyrosine kinases such as src, fyn and many others known in the art. These peptides are of the formula

FL-A-pY-TGLSTRNQET-pY-ATH-NH$_2$

FL-pY-pY-pY-IE-NH$_2$

FL-G-pY-NELNLGRREE-pY-DVL-NH$_2$

Here, Y is the one-letter abbreviation for tyrosine, pY is phosphorylated tyrosine, and A, T, G ... are one-letter abbreviations for other amino acids, as specified in the Appendix.

Preferred embodiments for FL include various FL-S moieties, especially the 5-carboxy and 6-carboxy derivatives of fluorescein, which readily form amides at the N-terminus of the peptide.

These peptides are suitable tracers for competition assays with respect to analytes that contain phosphorylated tyrosine, such as the activated insulin receptor and natural or artificial substrates of it. They are advantageous over peptide labels known in the prior art by virtue of their enhanced affinity for specific binding partners for phosphorylated peptides containing phosphotyrosines. The assay methods may be extended to phosphoserine/threonine as well.

The peptides are thus useful as tracers in any luminescence polarization assay for phosphorylated peptides wherein the phosphorylation is associated with tyrosine residues. The peptides are added to a sample that contains or is suspected to contain such phosphorylated peptides along with an opposite member of a specific binding pair involving these peptides. The peptides compete with any phosphorylated protein in the sample for the opposite member so that the degree of luminescence polarization observed in light emitted from the sample is inversely correlated with the concentration of phosphorylated protein in the sample. This determination can also be made as a function of time if the activity of an enzyme that phosphorylates proteins is to be measured. In addition, the effect of compounds on such phosphorylation reactions can be studied by conducting the assay in the presence and in the absence of candidate compounds.

Example 9

Metal-Based Assays

The presence and activity of kinases and phosphatases can be determined using assays capable of detecting phosphorylated and dephosphorylated protein, as described above. These assays may involve monitoring changes (e.g., increases or decreases) in the interactions between a protein substrate and a binding partner capable of recognizing the phosphorylated substrate but not the dephosphorylated substrate, or vice versa. In particular, these assays may employ specific binding partners such as antibodies that recognize only particular phosphorylated or dephosphorylated substrates (such as a phosphorylated tyrosine, serine, or threonine surrounded by a particular sequence of amino acids). Thus, these assays typically require careful matching of the substrate and binding partner, potentially limiting the substrates to those for which a specific binding partner is available.

The assays described here may circumvent these limitations by permitting the use of relatively nonspecific binding partners in phosphorylation assays. Such binding partners may recognize phosphorylated protein substantially without regard to the particular phosphorylated amino acid or surrounding amino acid sequence. Examples of such binding partners include metal cations, and especially trivalent metal cations such as $Fe^{3+}$ (iron) and $Ga^{3+}$ (gallium). Examples also may include such binding partners complexed with other compositions, including macromolecules and/or surfaces. Potential difficulties with this approach include (1) interference from compounds (e.g., ATP, free phosphate, EDTA, and possibly primary/secondary amines) that may compete with or otherwise affect the interaction between the metal and the phosphorylated protein, and (2) difficulty in maintaining a pH that preserves the affinity and selectivity of the binding between the metal and phosphorylated protein.

Changes in the interactions between the protein substrate and (specific or nonspecific) binding partner brought about by enzyme activity may be monitored using any suitable assay format and technique. For example, changes may be monitored using competition or sandwich assays, and homogeneous or heterogeneous assays, including heterogeneous assays using plates or beads. Changes also may be monitored using luminescence-based techniques, including energy-transfer and polarization-based techniques. Changes also may be monitored using microfluidics-based techniques.

Further aspects of the invention are illustrated without limitation in the following examples:

Example 9A

$Ru^{2+}$ was entrapped in a small (~20-30 kDa) synthetic polymer macromolecule (MM) obtained from Presens (Germany). The macromolecule is relatively hydrophilic, with carboxyl groups on its surface for activation. The MM with the entrapped $Ru^{2+}$ was used as a support to immobilize trivalent metal cations, including $Fe^{3+}$ and $Ga^{3+}$. A kinase

enzyme phosphorylated a luminescently labeled kinase substrate, which bound to the metal cations immobilized on the MM. Binding was detected using polarization and energy transfer techniques. Specifically, binding led to a decrease in substrate mobility and a concomitant increase in the polarization of light emitted by the luminophores bound to the substrate. Similarly, binding led to a decrease in separation between the luminophores bound to the substrate and the $Ru^{2+}$ immobilized in the MM, and a concomitant increase in energy transfer from the $Ru^{2+}$ (donor) to the luminophore (acceptor). Polarization and energy transfer can be detected using apparatus and methods described in patent applications listed in the introductory paragraphs.

This approach may be extended through various changes and modifications. For example, in polarization assays, the $Ru^{2+}$ is irrelevant and may be omitted, because the MM is just a carrier. In energy transfer assays, the approach may be extended to any energy transfer pair, including a lanthanide. Also, the luminescent donor need not be encapsulated in the MM. One could attach a luminescent species directly to a suitable $Fe^{3+}$ or $Ga^{3+}$ chelate. In a heterogeneous assay, phosphorylated proteins bound via $Ga^{3+}$ or $Fe^{3+}$ binding as above to microplates, particles, or inner surfaces of microfluidic devices may be detected after a wash by measuring luminescence intensity. Such detection can take place either directly on the surfaces or in the solution phase by adding an elution solution such as a phosphate buffer. With other detection methods, such as the laser-scanning method used in FMAT™ technology by PE Biosystems, the bound phosphoproteins can be detected directly on the beads, without the need for washing or separation. Other labels such as enzymes also may be used in the heterogeneous format.

## Example 9B

The chelator imidodiacetic (IDA) acid was linked to the MM using the secondary amine group of IDA and a carboxyl group on the MM. Afterwards, the MM-IDA was incubated with either $FeCl_3$ or $GaCl_3$. It was found that the $FeCl_3$ quenches the luminescence of Ru, while the $GaCl_3$ does not.

The macromolecule loaded with $Ga^{3+}$ (MM-Ga) was tested for the binding of the following di-(phosphotyrosine) peptide fluorescein-labeled tracer, denoted tyrosine kinase 1 (TK-1) tracer:

FL-A-pY-TGLSTRNQET-pY-ATH-NH$_2$.

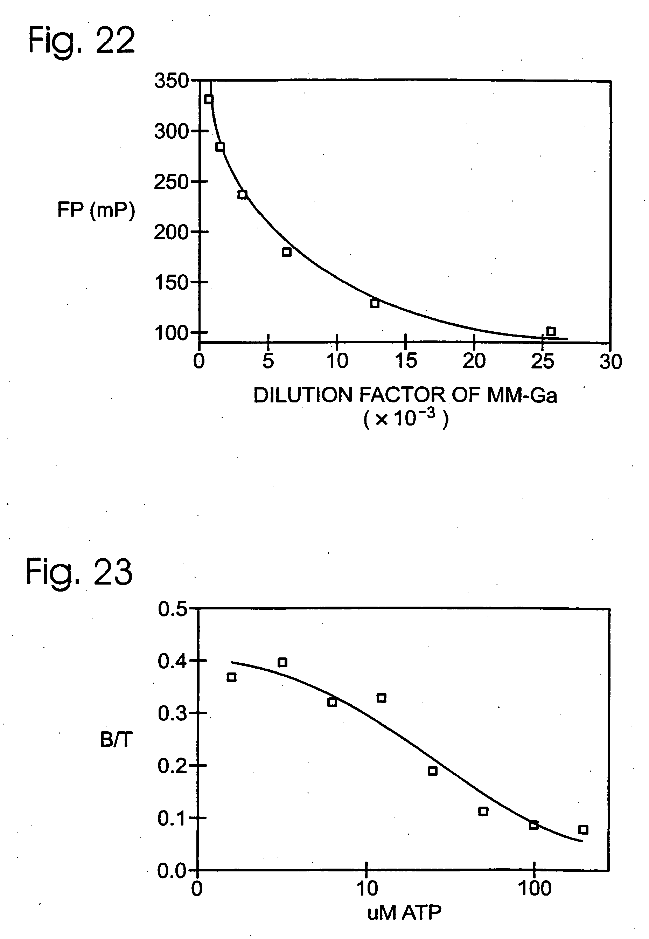

Figure 22 shows the effects of incubating 10 nM TK-1 tracer with different concentrations of MM-GA (total volume = 50 $\mu$L; incubation time = 60 minutes). The maximum polarization change was more then 200 mP when the MM-Ga and TK-1 tracer were incubated in MES buffer (0.1M MES, pH 5.5, 1.0 M NaCl).

Figure 23 shows a dose-response curve for ATP, with 10 nM TK-1 tracer and 1.6 nM (estimated) MM-Ga. The bound/total ratio of the tracer was calculated based on the measured luminescence polarization value. Here, the IC50 for ATP was around 10 $\mu$M, in an assay with 10 nM of tracer and 1:4000 dilution of MM-Ga (final concentration).

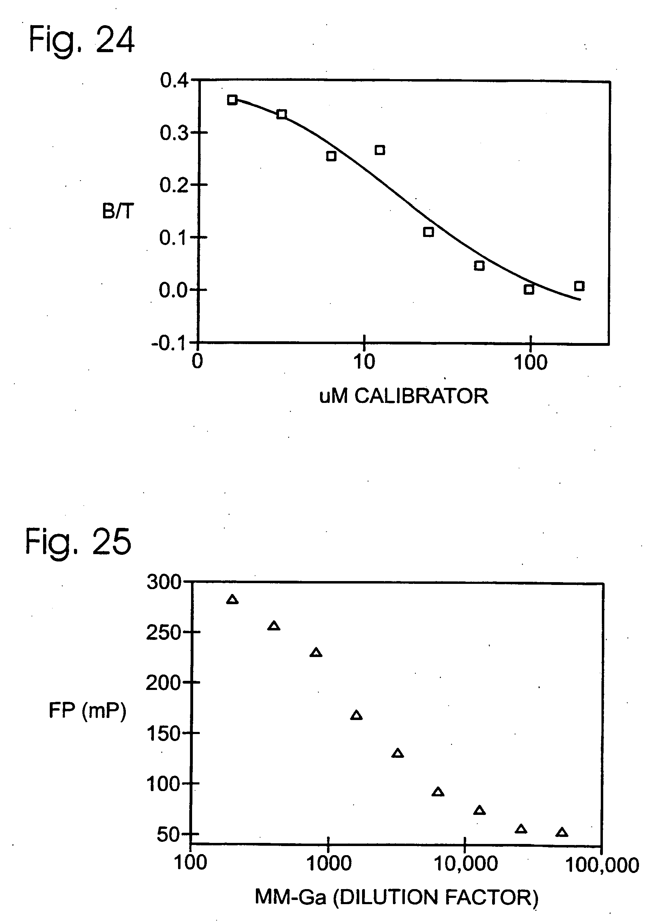

Figure 24 shows a dose-response curve for TK-1 calibrator, with 10 nM TK-1 tracer and 1.6 nM (estimated) MM-Ga. The TK-1 calibrator is the same phosphopeptide as TK-1 tracer without a fluorescein label. The bound/total ratio was calculated as in Figure 23. The IC50 for the TK-1 calibrator also was around 10 $\mu$M.

The macromolecule loaded with $Ga^{3+}$ (MM-Ga) also was tested for the binding of the following fluorescein-labeled monophosphoserine peptide tracer, denoted serine/threonine kinase 1 (STK-1) tracer: FL-RFARKG-pS-LRQKNV.

Figure 25 shows binding of STK-1 tracer to various concentrations of MM-Ga (STK-1 tracer concentration = 5 nM; total volume = 50 $\mu$L; incubation time = 70 minutes).

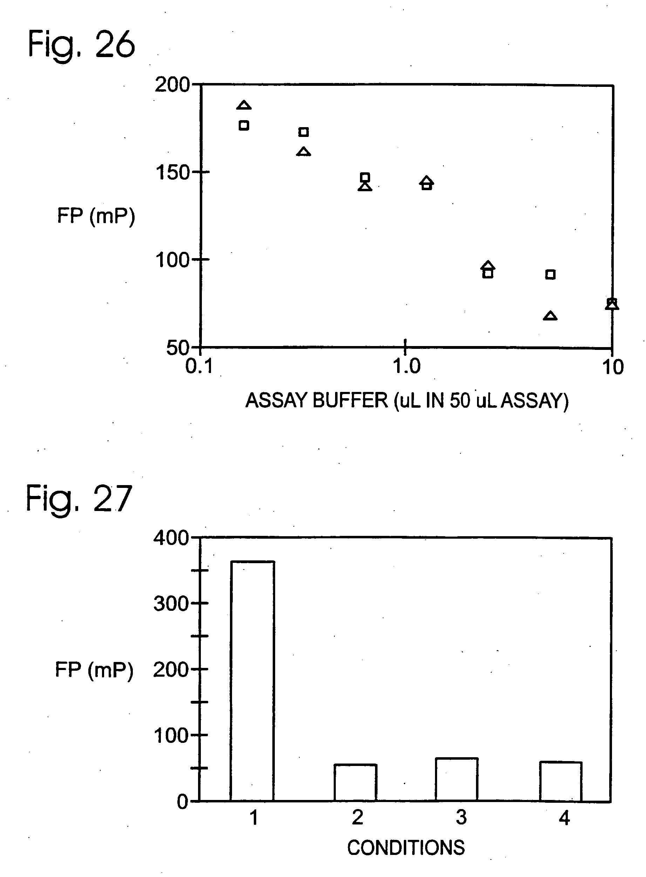

Figure 26 shows the effects of a HEPES-based buffer on the MM-Ga/phosphopeptide interaction (STK-1 tracer concentration = 5 nM; MM-Ga is diluted 1:1000). The buffer included 140 mM NaCl, 20 mM HEPES, and 0.1% bovine gamma globulin. The results show that such buffers interfere with the MM-Ga/tracer interaction.

## Example 9C

We demonstrated the specificity of the binding between phosphorylated peptide and a macromolecule activated with Ga (III) metal ion (MM-Ga) and its potential in the development of generic kinase assays. The materials included cAMP-dependent protein kinase A (PKA, from Promega) as the enzyme and fluorescein-labeled Kemptide (fluo-Leu-Arg-Arg-Ala-Ser-Leu-Gly) as the substrate.

(1) Endpoint assay. Figure 27 shows an endpoint assay of PKA activity with MM-Ga under the following conditions: (1) reaction with enzyme, assay with MM-Ga; (2) reaction with enzyme, no MM-Ga; (3) no enzyme control, with MM-Ga; and (4) no enzyme control, no MM-Ga. The assay was performed as follows. First, a mixture was prepared of 20 mM $MgCl_2$, 0.2 mM ATP, 2 mM $NaVO_4$, and 100 $\mu$M fluo-Kemptide in a total of 50 $\mu$lL 40 mM Tris-HCl, pH 7.4. Second, the reaction was initiated by adding 1.0 $\mu$L of the enzyme PKA to the mixture; for a control reaction, no PKA was added. Third, the reaction was run overnight at room temperature. Fourth, the reaction and control were diluted 1:1000, and 1 µL of the diluted solution was added to a volume of 49 µL of MM-Ga solution (approx. 30 nM MM-Ga) in a MES buffer (pH 5.5) in a 384-well plate. Fifth, the plate was incubated at room temperature for 60 min. Finally, the luminescence polarization was measured using an LJL Analyst™ light-detection platform.

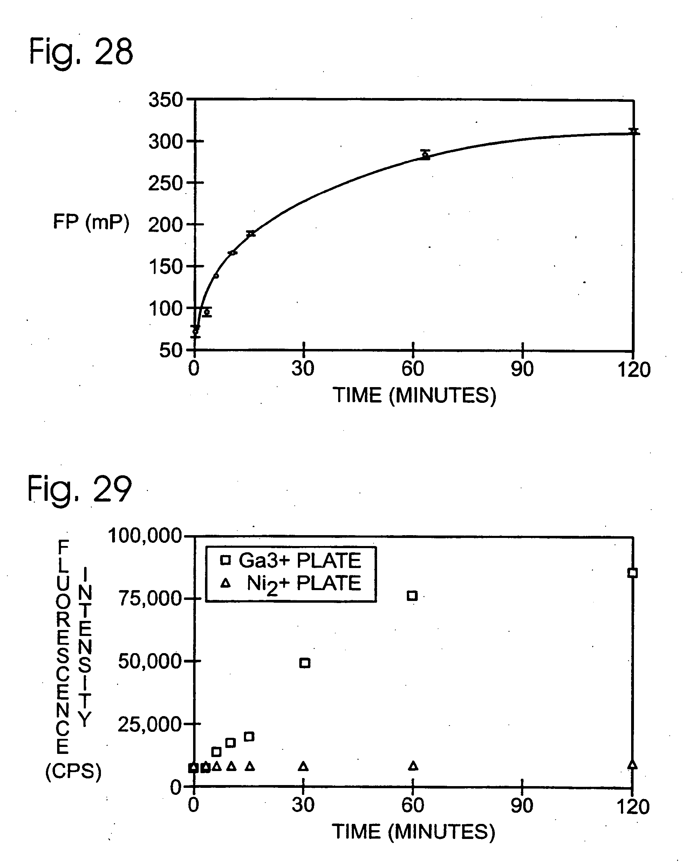

(2) <u>Time-course assay</u>. Figure 28 shows a time-course assay of PKA activity with MM-Ga performed under the reaction conditions of Figure 27. At preselected times, 1 µL of reaction mixture was taken out from the reaction and immediately diluted into a volume 1000 µL of MES buffer. Afterwards, 1 µL of each diluted sample was added to a volume of 49 µL of MM-Ga solution, and an assay was conducted as described above.

The results show that phosphorylated peptide binds specifically to MM-Ga. Figures 27 and 28 show that MM-Ga does not bind to non-phosphorylated Kemptide but binds strongly to phosphorylated Kemptide. Figure 28 shows the potential of using the MM-Ga in a kinase reaction.

Example 9D

The metal ions (e.g., $Ga^{3+}$) immobilized on the macromolecules in the PKA/Kemptide system can bind selectively to phosphorylated peptide generated in a kinase reaction. Using the IMAP (immobilized metal with affinity to phosphoproteins) system, we were able to monitor the time course of a kinase reaction using a homogeneous luminescence polarization (FP) assay. In particular, the polarization changed from 50 mP before the reaction was started to 300 mP after the reaction was completed.

Alternatively, the IMAP technology can be used in a heterogeneous assay format with a metal-coated plate. Here, the feasibility of using metal-coated plates in the development of generic kinase assays is demonstrated with a commercial $Ni^{2+}$-coated plate, in which the $Ni^{2+}$ has been replaced with $Ga^{3+}$. 200 µL 0.5 M EDTA-containing solution was added to each well of a 96-well $Ni^{2+}$-coated plate, and the plate was incubated at room temperature for 1 hour. The process was repeated two more times to remove all of the $Ni^{2+}$ from the plate. The plate was then washed 3 times with 10 mM Tris buffer, pH 7.4. Next, 200 µL 0.1 M $GaCl_3$ solution was added to the each well of the plate, and the plate was incubated at room temperature for overnight. The plate was washed three times before being used in a kinase assay.

In the assay, a kinase reaction was set up as previously described, using PKA as the enzyme and fluorescein-Kemptide as the substrate. At each time point, 1 µL was taken from the reaction and diluted into 1000 µL of MES buffer. Later, a volume of 100 µL of each diluted solution was added to the $Ga^{3+}$-coated plate, and incubated for 1 hour at room temperature. The plate was then washed three times, and 100 µL of a 1 M $KH_2PO_4$ solution was added to elute the bound phosphorylated Kemptide from the plate. The fluorescence intensity was measured with an LJL Analyst™ light-detection platform set in fluorescence intensity mode and using a medium attenuator. In a control assay, an unmodified $Ni^{2+}$-coated plate was used. The results are shown in Figure 29.

Although this is a heterogeneous assay format, which requires several washing steps, it offers the advantages of a generic assay that is applicable in principle to any kinase regardless of its substrate specificity. This may save assay developers 3 to 6 months of time and effort in making antibodies that recognize specifically a phosphorylated version of an amino acid sequence. The lack of availability of such special antibodies often is the major obstacle in the development of non-radioactive kinase assays.

Thus, for high-throughput screening applications, there are several possible assay designs that take advantage of MM-Ga in the development of generic kinase assays. These assays do not rely on a specific anti-phosphoprotein antibody and are applicable to a wide variety of kinases regardless of their substrate specificity.

Example 10

Tyrosine-Kinase Assay With BODIPY-TMR Tracer

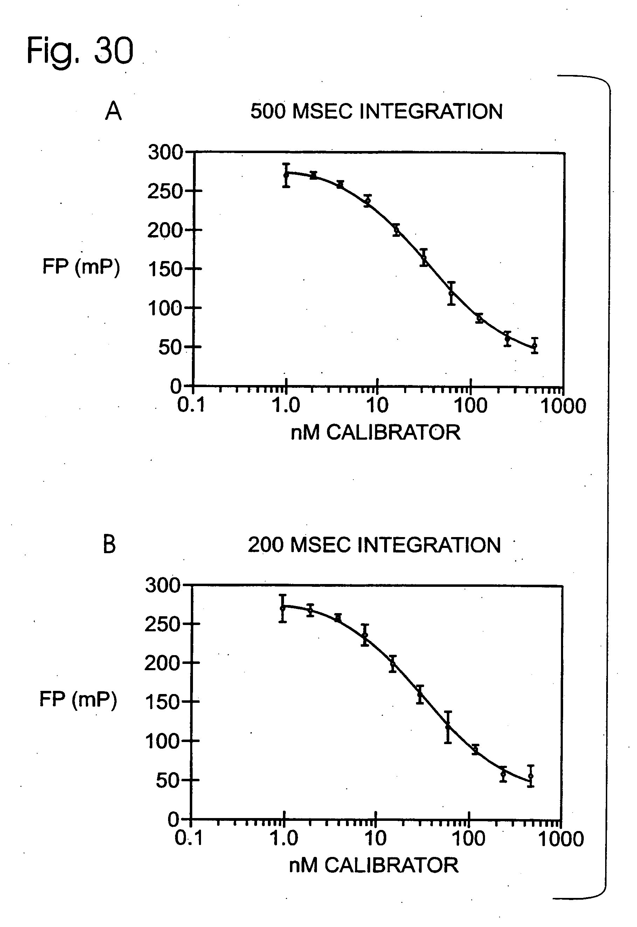

Figure 30 shows polarization calibration curves for TK-1 with BODIPY-TMR tracer, for (A) 500 msec and (B) 200 msec integration times. Experimental conditions were as follows:

Tracer = 0.4 nM TK-1 peptide, labeled with TMR-Bodipy (final)
Ab = 1:400 (1:1600 final)
1 hour incubation; 500 msec integration

Measured values of IC50 were 33 nM for (A) and 32 nM for (B). Results are essentially the same as those obtained with a fluorescein label.

Example 11

Integrated Cell-signaling Assays

This example shows assays for integrated cell signaling mechanisms. Specifically, the assays described here for kinase and phosphatase enzymes may be performed in combination with assays for cyclic nucleotides and GTP-binding proteins. Such combination assays permit study of signaling mechanisms involving multiple pathways.

Example 12

Improved-signal Assay

This example shows assays with improved signals, signal-to-noise ratios, and/or signal-to-background ratios.
Signal may be enhanced in several ways, including (1) using a high color temperature light source, such as a xenon arc lamp, in a continuous illumination mode, (2) using a dichroic or multi-dichroic beamsplitter, and/or (3) using a sample holder whose shape is "matched" to the shape of the optical beam of the instrument, especially if the sample holder is elevated to bring the sample closer to a detector. The high color temperature light source increases the number of usable photons, which is important because the lower limit of the signal-to-noise ratio is set by the square root of the total number of photons collected in the measurement.
Signal-to-noise ratios can be enhanced at least in part by increasing signals, for example, by using the techniques described in the previous paragraph.
Signal-to-background ratios can be enhanced in several ways, including (1) using confocal optical systems having a sensed volume to avoid luminescence from the microplate walls, (2) selecting a microplate or other substrate that increases the signal and reduces the luminescent background from materials in the microplate, (3) selecting the light sources, luminescence filters, optics, signal collection electronics, and mechanical system used in the luminescence detection optical system for maximum signal-to-background ratio, and (4) utilizing signal processing, background subtraction, and luminescence lifetime techniques, particularly FLAMe™ methodology for background reduction, as described below. These enhancements are described in more detail in U.S. Patent No. 6,071,748.

Example 13

Improved-polarization Assays

This example shows mechanisms for increasing the change in polarization that accompanies a change in binding, so that the change in binding can be measured more easily. These mechanisms may be used in any of the assays described here involving luminescently labeled species, such as labeled peptide tracers, among others.
The change in polarization upon binding can be increased by making any linker between the luminophore and the labeled species (e.g., the peptide tracers) as short and/or rigid as possible, while maintaining relevant substrate properties for the enzymes involved in the assay. Short and/or rigid linkers will restrict luminophore motion relative to the labeled species, reducing the "propeller effect" so that the luminophore more accurately reports the motion of both the free and bound labeled species. The rigidity of the linker may be increased by avoiding using hexanoic acid linkers, which typically are long and flexible, and by using amide groups in place of methylene groups, among other mechanisms.
The change in polarization upon binding also can be increased by including an appropriately positioned energy transfer acceptor on the binding partner, so that energy transfer will occur from the luminophore to the acceptor upon incorporation. Such energy transfer will shorten the lifetime of the luminophore, thereby increasing its polarization (because polarization varies inversely with lifetime, all else being equal).
The change in polarization upon binding also can be increased by decreasing the mobility of the binding partner for the labeled species. Mobility can be decreased by increasing the size of the binding partner, either directly or by forming a complex with a mass label. Suitable mass labels include other molecules and beads, among others. The use of mass labels is described in detail in PCT Patent Application Serial No. PCT/US99/24707 (Publication No. WO/2000/023785). Mobility also can be decreased by attaching the binding partner to a surface, such as the surface of a sample holder. Attachment to other molecules, beads, and/or surfaces may be accomplished using any of a number of well-known reactive groups.

Example 14

Principles of Luminescence Polarization Assays

This example describes principles of luminescence polarization assays. Here, luminescence refers to the absorption and subsequent re-emission of light by a luminescent molecule, or "luminophore," and polarization refers to the direction of the light's electric field, which generally is perpendicular to the direction of the light's propagation. Luminescence includes fluorescence and phosphorescence, among others. In a luminescence polarization assay, specific molecules within a composition are labeled with one or more luminophores. The composition then is illuminated with polarized excitation light, which preferentially excites luminophores having absorption dipoles aligned parallel to the polarization of the excitation light. These molecules subsequently decay by preferentially emitting light polarized parallel to their emission dipoles. The extent of polarization of the total emitted light depends on the extent of molecular reorientation during the time interval between luminescence excitation and emission, which is termed the luminescence lifetime, $\tau$. In turn, the extent of molecular reorientation depends on the luminescence lifetime and the size, shape, and environment of the reorienting molecule. Thus, luminescence polarization assays may be used to quantify binding reactions and enzymatic activity, among other applications. In particular, molecules commonly rotate (or "tumble") via diffusion, with a rotational correlation time $\tau_{rot}$ that is proportional to their volume, or the cube of their radius of gyration. (This cubic dependence on radius makes polarization assays very sensitive to binding.) Thus, during their luminescence lifetime, relatively large molecules will not reorient significantly, so that their total luminescence will be relatively polarized. In contrast, during the same time interval, relatively small molecules will reorient significantly, so that their total luminescence will be relatively unpolarized.

The relationship between polarization and intensity is expressed by the following equation:

$$P = \frac{I_{\parallel} - I_{\perp}}{I_{\parallel} + I_{\perp}} \tag{1}$$

Here, $P$ is the polarization, $I_{\parallel}$ is the intensity of luminescence polarized parallel to the polarization of the excitation light, and $I\perp$ is the intensity of luminescence polarized perpendicular to the polarization of the excitation light. $P$ generally varies from zero to one-half for randomly oriented molecules (and zero and one for aligned molecules). If there is little rotation between excitation and emission, $I_{\parallel}$ will be relatively large, $I\perp$ will be relatively small, and $P$ will be close to one-half. (P may be less than one-half even if there is no rotation; for example, $P$ will be less than one-half if the absorption and emission dipoles are not parallel.) In contrast, if there is significant rotation between absorption and emission, $I_{\parallel}$ will be comparable to $I\perp$ and $P$ will be close to zero. Polarization often is reported in milli-P units ($1000 \times P$), which for randomly oriented molecules will range between 0 and 500, because $P$ will range between zero and one-half.

Polarization also may be described using other equivalent quantities, such as anisotropy. The relationship between anisotropy and intensity is expressed by the following equation:

$$r = \frac{I_{\parallel} - I_{\perp}}{I_{\parallel} + 2I_{\perp}} \tag{2}$$

Here, $r$ is the anisotropy. Polarization and anisotropy include the same information, although anisotropy may be more simply expressed for systems containing more than one luminophore. In the description and claims that follow, these terms may be used interchangeably, and a generic reference to one should be understood to imply a generic reference to the other.

The relationship between polarization and rotation is expressed by the Perrin equation:

$$\left( \frac{1}{P} - \frac{1}{3} \right) = \left( \frac{1}{P_0} - \frac{1}{3} \right) \cdot \left( 1 + \frac{\tau}{\tau_{rot}} \right) \tag{3}$$

Here, $P_0$ is the polarization in the absence of molecular motion (intrinsic polarization), $\tau$ is the luminescence lifetime (inverse decay rate) as described above, and $\tau_{rot}$ is the rotational correlation time (inverse rotational rate) as described above.

The Perrin equation shows that luminescence polarization assays are most sensitive when the luminescence lifetime and the rotational correlation time are similar. Rotational correlation time is proportional to molecular weight, increasing by about 1 nanosecond for each 2,400 dalton increase in molecular weight (for a spherical molecule). For shorter lifetime luminophores, such as fluorescein, which has a luminescence lifetime of roughly 4 nanoseconds, luminescence polarization assays are most sensitive for molecular weights less than about 40,000 daltons. For longer lifetime probes, such as Ru(bpy)$_2$dcbpy (ruthenium 2,2'-dibipyridyl 4,4'-dicarboxyl-2,2'-bipyridine), which has a lifetime of roughly 400 nanoseconds, luminescence polarization assays are most sensitive for molecular weights between about 70,000 daltons and 4,000,000 daltons.

Luminescence polarization assays may be used in a variety of formats. In one format, the concentration of an analyte in solution can be measured by supplying a labeled tracer that competes with the analyte for a binding moiety, particularly a binding moiety larger than the labeled tracer. In this "competitive" format, the concentration of the analyte is inversely correlated with the enhancement of luminescence polarization in the light emitted by the tracer when it competitively binds the common moiety. In another format, the concentration of a target can be measured by supplying a labeled tracer that is capable of binding the target. In this case, the enhancement of polarization is a direct measure of the concentration of target. The target further may be, for example, an activated receptor, where activation can be indirectly measured by the directly measured concentration of a generated molecule or by its binding to labeled tracer *per se*.

### **Appendix**

The following table lists the one- and three-letter abbreviations for the twenty amino acids commonly found in biological systems.

| Amino acid | Three-letter abbreviation | One-letter abbreviation |
|---|---|---|
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamic acid | Glu | E |
| Glutamine or glutamic acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

### Claims

1. A method of detecting addition or removal of a phosphate group to or from a substrate comprising
contacting a luminescent peptide with a binding partner that binds specifically to a phosphorylated peptide substantially without regard to the particular amino acid sequence of the peptide, wherein the peptide is a substrate for an enzyme that catalyzes addition or cleavage of a phosphate group to or from a protein, and
measuring luminescence polarization from the luminescent peptide, wherein the amount of measured luminescence polarization can be related to the extent of binding between the luminescent peptide and the binding partner.

2. The method of claim 1 further comprising the step of correlating luminescence polarization with kinase or phosphatase activity.

3. A method of detecting addition or removal of a phosphate group to or from a substrate comprising contacting a luminescent peptide with a binding partner that binds specifically to a phosphorylated peptide substantially without regard to the particular amino acid sequence of the peptide, wherein the peptide is a substrate for an enzyme that catalyzes addition or cleavage of a phosphate group to or from a protein, and
measuring luminescence resonance energy transfer (FRET), and relating FRET to the extent of binding between the luminescent peptide and the binding partner.

4. The method of claim 3 further comprising the step of correlating FRET with kinase or phosphatase activity.

5. The method of any preceding claim, wherein the peptide is amidated on one end.

6. The method of any preceding claim, wherein the binding partner includes a metal cation that binds specifically to the peptide only when the peptide is a phosphorylated peptide.

7. The method of claim 6, wherein the binding partner also comprises ruthenium.

8. The method of claim 6 or 7, wherein the macromolecule has a molecular weight between about 20 and 30 kilodaltons.

9. The method of any of claims 6 to 8, wherein the metal cation comprises gallium.

10. The method of any preceding claim, wherein the protein and the peptide are the same.

11. The method of any preceding claim, wherein the protein and the peptide are different.

12. The method of any preceding claim further comprising the step of providing at least one phosphate group on the luminescent peptide, and competing with the luminescent peptide by catalyzing formation of unlabelled phosphorylated protein.

13. The method of any preceding claim, wherein the peptide can be phosphorylated by one or more of the following enzymes: serine/threonine kinase, threonine/tyrosine kinase, and tyrosine kinase.

14. The method of any preceding claim, wherein the luminescent peptide includes at least one amino acid selected from the group consisting of serine and threonine.

15. The method of claim 14, wherein the serine and threonine is either near a sequence that is enriched with basic amino acids, or followed in the C- terminal direction by a proline residue.

16. The method of claim 15, wherein the basic amino acids are selected from the group consisting of lysine, arginine, and histidine.

17. The method of claim 15 or 16, wherein the serine or threonine is followed within three amino acids in the C-terminal direction by a basic amino acid.

18. The method of any of the claims 15 to 17, wherein the serine or threonine is followed immediately in the C-terminal direction by a proline residue.

19. The method of any preceding claim, wherein the luminescent peptide has fewer than about 15 amino acids.

20. The method of any of claims 1 to 12, wherein the peptide is selected from the group consisting of

ApYTGLSTRNQETpYATH-NH$_2$,

pYpYpYIE-NH$_2$,

GpYNELNLGRREEpYDVL-NH$_2$,

EKRPpSRSKYL,

EKRPSQRpSYL,

EKRPpSRpSYL,

KRREILSRRPpSYRK,

KHFPQFpSYSAS,

pSPELERLIIQC,

GSPSVRCSpSMpS,

RSRHSpSYPAGT,

LpTPLK,

FpTPLQ,

RKRpTLRRL,

LRRApSLG,

KKLNRTLpSVASL,

RPRAApTF-NH$_2$,

and

LRRApSLG-NH$_2$.

21. The method of any preceding claim, further comprising illuminating the sample with polarized light from a high color temperature continuous light source.

22. A method to assess the ability of a candidate compound to enhance or inhibit phosphorylation in a substrate protein which method comprises
performing the method of any preceding claim in the presence and absence of said candidate compound, and determining the degree of polarization or, as the case may be, the luminescence resonance energy transfer (FRET) in the presence as compared to the absence of said candidate compound, whereby an increase in the degree of measured polarization or, as the case may be, the luminescence resonance energy transfer (FRET) in the presence as opposed to the absence of said candidate compound identifies said compound as an inhibitor of said phosphorylation, and whereby a decrease in the degree of polarization or, as the case may be, the luminescence resonance energy transfer (FRET) in the presence as compared to the absence of said compound identifies said compound as an enhancer of said phosphorylation.

**Patentansprüche**

1. Verfahren zum Erfassen der Hinzufügung oder Entfernung einer Phosphatgruppe zu beziehungsweise von einem Substrat, welches folgende Schritte umfasst:

EP 1 261 621 B1

Inkontaktbringen eines lumineszenten Peptids mit einem Bindungspartner, welcher sich spezifisch an ein phosphoryliertes Peptid im Wesentlichen unabhängig von der besonderen Aminosäurensequenz des Peptids bindet, wobei das Peptid ein Substrat für ein Enzym ist, welches die Hinzufügung oder Abspaltung einer Phosphatgruppe zu beziehungsweise von einem Protein katalysiert, und

Messen der Lumineszenzpolarisation von dem lumineszenten Peptid, wobei das Ausmaß der gemessenen Lumineszenzpolarisation in Bezug zu dem Grad der Bindung zwischen dem lumineszenten Peptid und dem Bindungspartner gebracht werden kann.

2. Verfahren nach Anspruch 1, welches ferner folgenden Schritt umfasst:

Herstellen eines Bezugs zwischen der Lumineszenzpolarisation und der Kinase- oder Phosphataseaktivität.

3. Verfahren zum Erfassen der Hinzufügung oder Entfernung einer Phosphatgruppe zu beziehungsweise von einem Substrat, welches folgende Schritte umfasst:

Inkontaktbringen eines lumineszenten Peptids mit einem Bindungspartner, welcher sich spezifisch an ein phosphoryliertes Peptid im Wesentlichen unabhängig von der besonderen Aminosäurensequenz des Peptids bindet, wobei das Peptid ein Substrat für ein Enzym ist, welches die Hinzufügung oder Abspaltung einer Phosphatgruppe zu beziehungsweise von einem Protein katalysiert, und

Messen des Lumineszenz-Resonanz-Energietransfers (FRET) und Herstellung eines Bezugs zwischen dem FRET und dem Grad der Bindung zwischen dem lumineszenten Peptid und dem Bindungspartner.

4. Verfahren nach Anspruch 3, welches ferner folgenden Schritt umfasst:

Herstellen eines Bezugs zwischen dem FRET und der Kinase-oder Phosphataseaktivität.

5. Verfahren nach einem beliebigen vorangehenden Anspruch, wobei das Peptid an einem Ende amidiert ist.

6. Verfahren nach einem beliebigen vorangehenden Anspruch, wobei der Bindungspartner ein Metallkation enthält, welches sich spezifisch an das Peptid nur bindet, wenn das Peptid ein phosphoryliertes Peptid ist.

7. Verfahren nach Anspruch 6, wobei der Bindungspartner auch Ruthenium umfasst.

8. Verfahren nach Anspruch 6 oder 7, wobei das Makromolekül ein Molekulargewicht zwischen etwa 20 und 30 Kilodalton aufweist.

9. Verfahren nach einem beliebigen der Ansprüche 6 bis 8, wobei das Metallkation Gallium umfasst.

10. Verfahren nach einem beliebigen vorangehenden Anspruch, wobei das Protein und das Peptid gleich sind.

11. Verfahren nach einem beliebigen vorangehenden Anspruch, wobei das Protein und das Peptid unterschiedlich sind.

12. Verfahren nach einem beliebigen vorangehenden Anspruch, welches ferner folgenden Schritt umfasst:

Vorsehen mindestens einer Phosphatgruppe an dem lumineszenten Peptid und Konkurrieren mit dem lumineszenten Peptid durch Katalysieren der Bildung unmarkierten phosphorylierten Proteins.

13. Verfahren nach einem beliebigen vorangehenden Anspruch, wobei das Peptid durch eines oder mehr der folgenden Enzyme phosphoryliert werden kann: Serin-/Threonin-Kinase, Threonin-/ Tyrosin-Kinase und Tyrosin-Kinase.

14. Verfahren nach einem beliebigen vorangehenden Anspruch, wobei das lumineszente Peptid mindestens eine Aminosäure enthält, die aus der Gruppe ausgewählt wird, welche aus Serin und Threonin besteht.

15. Verfahren nach Anspruch 14, wobei sich das Serin und Threonin entweder nahe einer Sequenz befindet, welche mit basischen Aminosäuren angereichert ist, oder in Richtung des C-Terminus ein Prolinrest auf dieses folgt.

16. Verfahren nach Anspruch 15, wobei die basischen Aminosäuren aus der Gruppe ausgewählt werden, welche aus Lysin, Arginin und Histidin besteht.

24

**17.** Verfahren nach Anspruch 15 oder 16, wobei auf das Serin oder Threonin innerhalb von drei Aminosäuren in Richtung des C-Terminus eine basische Aminosäure folgt.

**18.** Verfahren nach einem beliebigen der Ansprüche 15 bis 17, wobei auf das Serin oder Threonin unmittelbar in Richtung des C-Terminus ein Prolinrest folgt.

**19.** Verfahren nach einem beliebigen vorangehenden Anspruch, wobei das lumineszente Peptid weniger als etwa 15 Aminosäuren aufweist.

**20.** Verfahren nach einem der Ansprüche 1 bis 12, wobei das Peptid aus der Gruppe ausgewählt wird, welche besteht aus:

ApYTGLSTRNQETpYATH-NH$_2$,

pYpYpYIE-NH$_2$,

GpYNELNLGRREEpYDVL-NH$_2$,

EKRPpSRSKYL,

EKRPSQRpSYL,

EKRPpSRpSYL,

KRREILSRRPpSYRK,

KHFPQFpSYSAS,

pSPELERLIIQC,

GSPSVRCSpSMpS,

RSRHSpSYPAGT,

LpTPLK,

FpTPLQ,

RKRpTLRRL,

LRRApSLG,

KKLNRTLpSVASL,

RPRAApTF-NH$_2$,

und

LRRApSLG-NH$_2$.

**21.** Verfahren nach einem beliebigen vorangehenden Anspruch, welches ferner folgenden Schritt umfasst:

Anstrahlen der Probe mit polarisiertem Licht von einer Quelle kontinuierlichen Lichts mit hoher Farbtemperatur.

**22.** Verfahren zur Bewertung der Fähigkeit eines Verbindungskandidaten, die Phosphorylierung bei einem Proteinsubstrat zu verstärken oder zu hemmen, wobei dieses Verfahren folgende Schritte umfasst:

Durchführen des Verfahrens nach einem beliebigen vorangehenden Anspruch in Anwesenheit und in Abwesenheit des genannten Verbindungskandidaten, und

Bestimmung des Polarisierungsgrades oder gegebenenfalls des Lumineszenz-Resonanz-Energietransfers (FRET) in Anwesenheit des genannten Verbindungskandidaten im Vergleich den in dessen Abwesenheit ermittelten Werten, wobei eine Erhöhung des gemessenen Polarisierungsgrades oder gegebenenfalls des Lumineszenz-Resonanz-Energietransfers (FRET) in Anwesenheit des genannten Verbindungskandidaten, im Gegensatz zu den in dessen Abwesenheit ermittelten Werten, die genannte Verbindung als Hemmer der genannten Phosphorylierung identifiziert und wobei eine Verringerung des gemessenen Polarisierungsgrades oder gegebenenfalls des Lumineszenz-Resonanz-Energietransfers (FRET) in Anwesenheit des genannten Verbindungskandidaten, im Gegensatz zu den in dessen Abwesenheit ermittelten Werten, die genannte Verbindung als Verstärker der genannten Phosphorylierung identifiziert.

**Revendications**

1. Procédé de détection de l'addition ou de l'élimination d'un groupe phosphate sur ou à partir d'un substrat comprenant

   - la mise en contact d'un peptide luminescent avec un partenaire de liaison qui se lie de façon spécifique à un peptide phosphorylé sensiblement sans tenir compte de la séquence particulière d'acides aminés du peptide, le peptide étant un substrat pour une enzyme qui catalyse une addition ou un clivage d'un groupe phosphate sur ou à partir d'une protéine ; et
   - la mesure d'une polarisation de luminescence à partir du peptide luminescent, la quantité de polarisation de luminescence mesurée pouvant être corrélée à l'étendue de la liaison entre le peptide luminescent et le partenaire de liaison.

2. Procédé selon la revendication 1, comprenant en outre l'étape de corrélation de la polarisation de luminescence avec une activité kinase ou phosphatase.

3. Procédé de détection de l'addition ou de l'élimination d'un groupe phosphate sur ou à partir d'un substrat, comprenant

   - la mise en contact d'un peptide luminescent avec un partenaire de liaison qui se lie de façon spécifique à un peptide phosphorylé sensiblement sans tenir compte de la séquence particulière d'acides aminés du peptide, le peptide étant un substrat pour une enzyme qui catalyse une addition ou un clivage d'un groupe phosphate sur ou à partir d'une protéine ; et
   - la mesure d'un transfert d'énergie de résonance de luminescence (FRET), et la corrélation du FRET à l'étendue de liaison entre le peptide luminescent et le partenaire de liaison.

4. Procédé selon la revendication 3, comprenant en outre l'étape de corrélation du FRET avec l'activité kinase ou phosphatase.

5. Procédé selon l'une quelconque des revendications précédentes, dans lequel le peptide est amidé sur une extrémité.

6. Procédé selon l'une quelconque des revendications précédentes, dans lequel le partenaire de liaison comprend un cation métallique qui se lie de façon spécifique au peptide seulement lorsque le peptide est un peptide phosphorylé.

7. Procédé selon la revendication 6, dans lequel le partenaire de liaison comprend également du ruthénium.

8. Procédé selon l'une des revendications 6 ou 7, dans lequel la macromolécule a une masse moléculaire entre environ 20 et 30 kilodaltons.

9. Procédé selon l'une quelconque des revendications 6 à 8, dans lequel le cation métallique comprend du gallium.

10. Procédé selon l'une quelconque des revendications précédentes, dans lequel la protéine et le peptide sont identiques.

11. Procédé selon l'une quelconque des revendications précédentes, dans lequel la protéine et le peptide sont différents.

12. Procédé selon l'une quelconque des revendications précédentes, comprenant en outre l'étape de disposition d'au moins un groupe phosphate sur le peptide luminescent, et de compétition avec le peptide luminescent par catalyse de la formation d'une protéine phosphorylée non marquée.

**13.** Procédé selon l'une quelconque des revendications précédentes, dans lequel le peptide peut être phosphorylé par une ou plusieurs des enzymes suivantes: sérine/thréonine kinase, thréonine/tyrosine kinase et tyrosine kinase.

**14.** Procédé selon l'une quelconque des revendications précédentes, dans lequel le peptide luminescent comprend au moins un acide aminé choisi dans le groupe constitué par la sérine et la thréonine.

**15.** Procédé selon la revendication 14, dans lequel la sérine et la thréonine sont soit près d'une séquence qui est enrichie en acides aminés basiques soit suivies dans la direction C-terminale par un résidu de proline.

**16.** Procédé selon la revendication 15, dans lequel les acides aminés basiques sont choisis dans le groupe constitué par la lysine, l'arginine et l'histidine.

**17.** Procédé selon l'une des revendications 15 ou 16, dans lequel la sérine ou la thréonine est suivie à l'intérieur de trois acides aminés dans la direction C-terminale par un acide aminé basique.

**18.** Procédé selon l'une quelconque des revendications 15 à 17, dans lequel la sérine ou la thréonine est suivie immédiatement dans la direction C-terminale par un résidu de proline.

**19.** Procédé selon l'une quelconque des revendications précédentes, dans lequel le peptide luminescent a moins d'environ 15 acides aminés.

**20.** Procédé selon l'une quelconque des revendications 1 à 12, dans lequel le peptide est choisi dans le groupe constitué par

ApYTGLSTRNQETpYATH-NH$_2$,

pYpYpYIE-NH$_2$,

GpYNELNLGRREEpYDVL-NH$_2$,

EKRPpSRSKYL,

EKRPSQRpSYL,

EKRPpSRpSYL,

KRREILSRRPpSYRK,

KHFPQFpSYSAS,

pSPELERLIIQC,

GSPSVRCSpSMpS,

RSRHSpSYPAGT,

LpTPLK,

FpTPLQ,

RKRpTLRRL,

LRRApSLG,

KKLNRTLpSVASL,

RPRAApTF-NH$_2$,

et

LRRApSLG-NH$_2$.

21. Procédé selon l'une quelconque des revendications précédentes, comprenant en outre l'éclairage de l'échantillon avec une lumière polarisée à partir d'une source de lumière continue à température de couleur élevée.

22. Procédé d'évaluation de l'aptitude d'un composé candidat à favoriser ou empêcher une phosphorylation dans une protéine de substrat, lequel procédé comprend:

- la réalisation du procédé selon l'une quelconque des revendications précédentes, en présence et en l'absence dudit composé candidat ; et
- la détermination du degré de polarisation ou, le cas échéant, le transfert d'énergie de résonance en luminescence (FRET) en présence par comparaison avec en l'absence dudit composé candidat, ce par quoi une augmentation du degré de polarisation mesuré ou, selon le cas échéant, le transfert d'énergie de résonance de luminescence (FRET) en présence par opposition avec en l'absence dudit composé candidat identifie ledit composé comme un inhibiteur de ladite phosphorylation, et ce par quoi une diminution du degré de polarisation ou, le cas échéant, le transfert d'énergie de résonance de luminescence (FRET) en présence par comparaison avec en l'absence dudit composé identifie ledit composé comme un activateur de ladite phosphorylation.

# Fig. 1

SIGNALING CELLS  SIGNAL SUBSTANCES  TARGET CELLS

# Fig. 2

G-PROTEIN-LINKED RECEPTOR MECHANISM

# Fig. 3

ENZYME-LINKED RECEPTOR MECHANISM

Fig. 4

INTRACELLULAR SIGNALING PATHWAYS

A. PHOSPHORYLATION PATHWAY

B. GTP-BINDING PATHWAY

# Fig. 5

# Fig. 6

FLUORESCENCE POLARIZATION DISPLACEMENT ASSAY

POLARIZATION OUTPUT

# Fig. 7

# Fig. 8

## Fig. 9

## Fig. 10

PHOSPHORYLATION OF SUBSTRATE
PEPTIDE BY IR-CKD

## Fig. 11

**TIME COURSE FOR THE EFFECT OF PRESENCE OF IR-CKD IN PHOSPHORYLATION OF "S"-PEPTIDE**

## Fig. 12

**34406711 INHIBITORY EFFECT OF PICEATANOL ON IR-CKD IN PHOSPHORYLATIONN OF "S"-PEPTIDE**

## Fig. 13

**SCREEN FOR AGONIST ACTIVITY USING IR-CKD**

% MAXIMAL POLARIZATION

ug/mL TER16998

## Fig. 14

**PEPTIDE**

% MAXIMAL POLARIZATION

Legend:
- ◇ IR-CKD
- ☐ IGF1r-CKD

uL REACTION

## Fig. 15

## Fig. 16

pY PEPTIDE MAPPING: PHOSPHORYLATION
DETECTION BY FLUORESCENCE POLARIZATION

SEPARATED HPLC FRACTIONS OF PEPTIDES
FROM DIGESTION OF PHOSPHORYLATED IR-CKD

## Fig. 17

EP 1 261 621 B1

## Fig. 18

**AB - TRACER COMPLEX**

## Fig. 19

**AB - TRACER COMPLEX**

## Fig. 20

## Fig. 21

Fig. 22

Fig. 23

## Fig. 24

## Fig. 25

Fig. 26

Fig. 27

## Fig. 28

## Fig. 29

# Fig. 30

**A**  500 MSEC INTEGRATION

**B**  200 MSEC INTEGRATION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 09160533 B
- US 6097025 A
- US 9901656 W
- WO 1999037203 A
- US 9903678 W
- WO 1999042817 A
- US 9908410 W
- WO 1999054711 A
- US 9916057 W
- WO 2000004364 A
- US 9916453 W
- WO 2000005336 A
- US 9916621 W
- WO 2000006991 A
- US 9916286 W
- WO 2000006989 A
- US 9916287 W
- WO 2000006990 A
- US 9924707 W
- WO 2000023785 A
- US 5235039 A
- US 4946778 A
- US 6071748 A
- US 9823095 W
- WO 1999023466 A

**Non-patent literature cited in the description**

- **K.E. van Holde.** Physical Biochemistry. 1985
- **William Bains.** Biotechnology from A to Z. 1993
- **Richard P. Haugland.** Handbook of Fluorescent Probes and Research Chemicals. 1996
- **Joseph R. Lakowicz.** Principles of Fluorescence Spectroscopy. 1999
- **Bob Sinclair.** Everything's Great When It Sits on a Chip: A Bright Future for DNA Arrays. *THE SCIENTIST,* 24 May 1999, vol. 13, 18
- **Charles R. Cantor ; Paul R. Schimmel.** *Biophysical Chemistry,* 1980
- **Jameson, D.M. et al.** Fluorescence Anisotropy Applied to Biomolecular Interactions. *Methods in Enzymology,* 1995, vol. 246, 283-300
- **Seethala, K.** *advance of the meeting of the Society for Bimolecular Screening,* 22 September 1997
- *Advances in Enzymology,* 1993, vol. 67, 265-299
- **Köhler ; Milstein.** *Nature,* 1975, vol. 256, 495
- **Kozbor et al.** *Immunology Today,* 1983, vol. 4, 72
- **Cole et al.** Monoclonal Antibodies and Cancer Therapy. Alan R. Liss, 1985, 77